(19) Europäisches Patentamt
European Patent Office
Office européen des brevets

(11) **EP 4 567 817 A1**

(12) **EUROPEAN PATENT APPLICATION**
published in accordance with Art. 153(4) EPC

(43) Date of publication:
**11.06.2025 Bulletin 2025/24**

(51) International Patent Classification (IPC):
***G16B 20/20*** (2019.01)

(21) Application number: 22953823.6

(22) Date of filing: **02.11.2022**

(86) International application number:
**PCT/CN2022/129143**

(87) International publication number:
**WO 2024/027032 (08.02.2024 Gazette 2024/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **01.08.2022 CN 202210914112**

(71) Applicant: **Guangzhou Burning Rock DX Co., Ltd. Guangzhou, Guangdong 510300 (CN)**

(72) Inventors:
• **LI, Bingsi**
  **Guangzhou, Guangdong 510300 (CN)**
• **XU, Jiayue**
  **Guangzhou, Guangdong 510300 (CN)**
• **QIU, Fujun**
  **Guangzhou, Guangdong 510300 (CN)**
• **HAN, Yusheng**
  **Guangzhou, Guangdong 510300 (CN)**
• **ZHANG, Zhihong**
  **Guangzhou, Guangdong 510300 (CN)**

(74) Representative: **Elzaburu S.L.P.
Paseo de la Castellana 259C
Torre de Cristal, planta 28
28046 Madrid (ES)**

(54) **METHOD AND SYSTEM FOR EVALUATING TUMOR FORMATION RISK AND TUMOR TISSUE SOURCE**

(57)     Provided are a tumor risk evaluation method and system. Specifically provided are a method and/or system for evaluating the correlation between a sample under test and a tumor formation risk and/or tumor tissue source. Methylation variation regions of various different cancers and specific methylation characteristic regions of various organs are captured by using DNA or RNA oligonucleotide sequences, the existence of tumor components (ctDNA) in blood cell-free DNA (cfDNA) is determined, and the correlation between the sample and the tumor tissue source is evaluated. Provided is a low-cost and high-accuracy method, which is conducive to accurately predicting and evaluating the risk of various cancers.

**Fig. 1**

**Description**

**Technical Field**

The present application relates to the field of biomedicine, in particular to a method and a system for evaluating tumor formation risk and tumor tissue source.

**Background**

DNA methylation is known to play an important role in the regulation of gene expression. Abnormal DNA methylation markers have been reported in the development of a variety of diseases, including cancer. DNA methylation sequencing is increasingly recognized as a high-resolution, high-throughput technique for cancer screening, diagnosis, and surveillance. WGBS (whole genome bisulfite sequencing) is the gold standard for methylation sequencing, but it is difficult for clinical application due to severe DNA damage and high sequencing cost during processing. More importantly, most regions of the human genome are inactive during cancer development, and cancer-related mutations tend to be centralized in specific regions, such as CpG islands, which provide a good opportunity for targeted sequencing.

However, the discovery and screening of Differentially Methylated Regions (DMRs) associated with cancer is challenging because population heterogeneity, including conditions such as disease or age, can bring non-specific changes in methylation patterns, so it is necessary to deal with these non-cancers but abnormal signals during the construction of DOC (Detection Of Cancer) model. Finally, for the application of detection for multiple cancer types, the establishment of Tissue Of Origin (TOO) model is of great auxiliary significance for tracing the possible source organs of cancer mutation, determining the downstream diagnosis and treatment path and saving healthcare costs.

**Summary**

The present application establishes a low-cost, high-precision method for capturing methylation variation regions of a variety of different cancers and specific methylation signature regions of various organs by using DNA or RNA oligonucleotide sequences, and determining the presence of tumor components (ctDNA) in circulating free DNA (cfDNA), and evaluating the correlation between samples and tumor tissue of origin.

In one aspect, the present application provides a method for evaluating correlation between a sample to be tested and risk of tumor formation and/or tumor tissue of origin, comprising: (1) a differentially methylated region DMR classification step: determining a plurality of target DMRs for evaluation, based on sequencing coverage depth of a methylated site and/or methylation level difference of adjacent methylated sites; (2) a tumor formation risk evaluation step: evaluating the correlation between the sample to be tested and the risk of tumor formation, based on methylation levels of the target DMRs of the sample to be tested; (3) optionally comprising a tumor tissue of origin evaluation step: evaluating the correlation between the sample to be tested and the tumor tissue of origin, based on methylation levels of the target DMRs of the sample to be tested.

In one aspect, the present application provides a method for determining a differentially methylated region DMR, the method comprising a differentially methylated region DMR classification step: determining a differentially methylated region DMR, based on sequencing coverage depth of a methylated site and/or methylation level difference of adjacent methylated sites.

In one aspect, the present application provides a method for evaluating correlation between a sample to be tested and risk of tumor formation, comprising a tumor formation risk evaluation step: evaluating the correlation between the sample to be tested and the risk of tumor formation, based on methylation level of the DMR of the sample to be tested, wherein the method comprising a step of reducing influences of age factor of a subject on evaluation result, wherein the sample to be tested is derived from the subject.

In one aspect, the present application provides a method for evaluating correlation between a sample to be tested and tumor tissue of origin, comprising a tumor tissue of origin evaluation step: evaluating the correlation between the sample to be tested and the tumor tissue of origin, based on methylation level of the DMR of the sample to be tested through a multi-classification method and logistic regression.

In one aspect, the present application provides a storage medium, recording a program capable of operating the method as described in the present application.

In one aspect, the present application provides an apparatus, the apparatus comprising the storage medium as described in the present application, and optionally comprising a processor coupled to the storage medium, and the processor being configured to implement the method as described in the present application by executing based on a program stored in the storage medium.

In one aspect, the present application provides a system for evaluating correlation between a sample to be tested and risk of tumor formation and/or tumor tissue of origin, characterized by comprising: (1) a differentially methylated region DMR classification module: used for determining a plurality of target DMRs for evaluation, based on sequencing coverage depth of a methylated site and/or methylation level difference of adjacent methylated sites; (2) a tumor formation risk evaluation module: used for evaluating the correlation between the sample to be tested and the risk of tumor formation, based on methylation levels of the target DMRs of the sample to be tested; (3) optionally comprising a tumor tissue of origin evaluation module: used for evaluating the correlation between the sample to be tested and the tumor tissue of origin, based on methylation levels of the target DMRs of the sample to be tested.

In one aspect, the present application provides a system used for determining a differentially methylated region DMR, characterized by comprising a differentially methylated region DMR classification module, used for determining a differentially methylated region DMR, based on sequencing coverage depth of a methylated site and/or methylation level difference of adjacent methylated sites.

In one aspect, the present application provides a system for evaluating correlation between a sample to be tested and risk of tumor formation, characterized by comprising a tumor formation risk evaluation module: used for evaluating the correlation between the sample to be tested and the risk of tumor formation, based on methylation level of the DMR of the sample to be tested, wherein the method comprising a module used for reducing the influence of age factor of a subject on evaluation result, wherein the sample to be tested is derived from the subject.

In one aspect, the present application provides a system for evaluating correlation between a sample to be tested and tumor tissue of origin, characterized by: comprising a tumor tissue of origin evaluation module: used for evaluating the correlation between the sample to be tested and the tumor tissue of origin, based on methylation level of the DMR of the sample to be tested through a multi-classification method and logistic regression.

The present application provides a low-cost, high-precision method, which is conducive to accurately predicting and evaluating the risk of multiple types of cancers.

Other aspects and advantages of the present application will be readily apparent to those skilled in the art from the following detailed description. Only exemplary embodiments of the present application are shown and described in the detailed description that follows. As those skilled in the art will recognize, the content of the present application enables those skilled in the art to make changes to the specific embodiments as disclosed without departing from the spirit and scope of the invention to which the present application relates. Accordingly, the drawings and descriptions in the specification of the present application are only exemplary and not to be limiting.

**Brief Description of the Drawings**

The application to which the present application relates is characterized in particular by the appended claims. The features and advantages of the invention to which the present application relates can be better understood by reference to the exemplary embodiments and drawings described in detail below. A brief description of the accompanying drawings is as follows:

Fig. 1 shows an exemplary scenario (a theoretical exemplary demonstration, not intended to represent actual sequencing situation).

Fig. 2 shows another exemplary scenario (a theoretical exemplary demonstration, not intended to represent actual sequencing situation).

Figs.3A-3C show another exemplary scenario (a theoretical exemplary demonstration, not intended to represent actual sequencing situation).

Fig. 4 shows that accuracy of 98% (95% CI: 96-99%) for tissue of origin can be achieved in 5-fold cross-validation.

Fig. 5 shows the results of controlling the weight allocation of confounding related features in the Salmon-DOC model of the present application.

Figs.6A-6F show that the Salmon-DOC model of the present application can efficiently detect 6 cancer types at different stages in the tumor group model.

Figs.7A-7F show that the Salmon-DOC model of the present application overcomes the weakness of the previous that false positives of methylation increasing with age in the healthy group and remains balanced across each age groups (age on the horizontal axis and cancer probability scores on the vertical axis).

Figs. 8A-8D show that the accuracy of origin of the Salmon-TOO two-layer model of the present application is better than that of the single-layer model in both cross-validation and independent validation.

Fig. 9 shows the results of the tissue of origin evaluation based on 103 TOO-related DMR regions.

## Detailed Description of the Embodiments

Embodiments of the invention of the present application are described below with reference to specific examples, and those skilled in the art will readily appreciate other advantages and effects of the invention of the present application from the disclosure of the present description.

## Definition of Terms

In the present application, the terms "second-generation gene sequencing (NGS)", "high throughput sequencing" or "next generation sequencing" generally refer to second generation high throughput sequencing technologies and higher throughput sequencing methods developed thereafter. Next-generation sequencing platforms include but are not limited to existing sequencing platforms such as Illumina. With the continuous development of sequencing technology, those skilled in the art can understand that sequencing methods and devices of other methods can also be used for the present methods. For example, second-generation gene sequencing may have the advantages of high sensitivity, large throughput, high sequencing depth, or low cost. According to the developmental history, influence, sequencing principle and technology, there are mainly the following types: Massively Parallel Signature Sequencing (MPSS), Polony Sequencing, 454 pyro sequencing, Illumina (Solexa) sequencing, Ion semi conductor sequencing, DNA nano-ball sequencing, and DNA nanoarray and combinatorial probe anchor ligation sequencing of Complete Genomics, etc. Such second-generation gene sequencing enables detailed overall analysis of the transcriptome and genome of a species, and is also known as deep sequencing. For example, the methods of the present application can also be applied to first-generation gene sequencing, second-generation gene sequencing, third-generation gene sequencing, or single-molecule sequencing (SMS).

In the present application, the term "sample to be tested" generally refers to a sample that needs to be tested. For example, one or more gene regions on the sample to be tested may be detected for the state of modification.

In the present application, the terms "polynucleotide", "nucleotide", "nucleic acid" and "oligonucleotide" can be used interchangeably. They present polymeric forms of nucleotides (deoxyribonucleotides or ribonucleotides) of any length, or analogs thereof. Polynucleotides can have any stereoscopic structure and can perform any function, whether known or unknown. The followings are non-limiting examples of polynucleotides: coding or non-coding regions of a gene or gene fragment, loci defined according to linkage analysis (loci), exons, introns, messenger RNA(mRNA), transfer RNA(tRNA), ribosomal RNA(rRNA), short interfering RNA(siRNA), short-hairpin RNA(shRNA), microRNA (miRNA), ribozymes, cDNA, recombinant polynucleotides, branched polynucleotides, plasmids, vectors, isolated DNA with any sequences, isolated RNA with any sequence, nucleic acid probes, primers and adapters. Polynucleotides may include one or more modified nucleotides, such as methylated nucleotides and nucleotide analogs.

In the present application, the term "methylation" generally refers to the methylation status possessed by a gene fragment, nucleotide or base thereof in the present application. For example, the DNA fragment in which the gene in the present application is located may have methylation on one strand or more strands. For example, the DNA fragment in which the gene in the present application is located may have methylation at one site or multiple sites.

In the present application, the term "human reference genome" generally refers to a human genome that can function as a reference in gene sequencing. For the information of the human reference genome, UCSC can be referenced to. The human reference genome may be in different versions, for example hg19, GRCH37 or ensembl 75.

In the present application, the term "machine learning model" generally refers to a system or set of program instructions and/or data being configured to implement an algorithm, process, or mathematical model. In the present application, the algorithm, process or mathematical model may evaluate and provide a desired output based on a given input. In the present application, the parameters of the machine learning model may not be explicitly programmed, and in the conventional sense, the machine learning model may not be explicitly designed to follow specific rules in order to provide a desired output for a given input. For example, the use of the machine learning model may mean that the machine learning model and/or the data structure/set of rules as the machine learning model is trained by a machine learning algorithm.

In the present application, the term "comprising" generally means including explicitly specified features but not excluding other elements.

In the present application, the term "about" generally refers to variations from a specified value in a range of above or below 0.5% - 10%, e.g., variations from a specified value in a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10%.

In order to realize that detection of six cancer types with high incidence and high lethality, which are: lung carcinoma, colorectal carcinoma, liver hepatocellular carcinoma, ovarian carcinoma, pancreatic adenocarcinoma, and esophageal carcinoma, the present application adopted a mode of combining a public database (TCGA) and internal data mining, adopted a novel algorithm, comparing methylation variation and spatial position of genomes simultaneously, and screened out 2536 differentially methylated regions (DMRs) highly correlated with cancer in total.

Summary

In one aspect, the present application provides a method for evaluating correlation between a sample to be tested and risk of tumor formation and/or tumor tissue of origin, which may comprise: (1) a differentially methylated region DMR classification step: determining a plurality of target DMRs for evaluation, based on sequencing coverage depth of a methylated site and/or methylation level difference of adjacent methylated sites; (2) a tumor formation risk evaluation step: evaluating the correlation between the sample to be tested and the risk of tumor formation, based on methylation levels of the target DMRs of the sample to be tested; (3) optionally comprising a tumor tissue of origin evaluation step: evaluating the correlation between the sample to be tested and the tumor tissue of origin, based on methylation levels of the target DMRs of the sample to be tested. For example, the method for evaluating the correlation between a sample to be tested and risk of tumor formation and/or tumor tissue of origin of the present application may comprise: (1) determining a plurality of target DMRs for evaluation, based on sequencing coverage depth of a methylated site and/or methylation level difference of adjacent methylated sites; (2) evaluating the correlation between the sample to be tested and the risk of tumor formation, based on methylation levels of the target DMRs of the sample to be tested; (3) optionally comprising evaluating the correlation between the sample to be tested and the tumor tissue of origin, based on methylation levels of the target DMRs of the sample to be tested..

In one aspect, the present application provides a method for determining a differentially methylated region DMR, the method may comprise a differentially methylated region DMR classification step: determining a differentially methylated region DMR, based on sequencing coverage depth of a methylated site and/or methylation level difference of adjacent methylated sites.

In one aspect, the present application provides a method for evaluating correlation between a sample to be tested and risk of tumor formation, comprising a tumor formation risk evaluation step: evaluating the correlation between the sample to be tested and the risk of tumor formation, based on methylation level of the DMR of the sample to be tested, wherein the method comprising a step of reducing influence of age factor of a subject on evaluation result, wherein the sample to be tested is derived from the subject.

In one aspect, the present application provides a method for evaluating correlation between a sample to be tested and tumor tissue of origin, comprising a tumor tissue of origin evaluation step: evaluating the correlation between the sample to be tested and the tumor tissue of origin, based on methylation level of the DMR of the sample to be tested through a multi-classification method and logistic regression.

For example, the method may comprise determining the DMR based on sequencing coverage depth of a methylated site and the methylation level difference between a methylated site and an adjacent methylated site thereof. For example, the methylation level difference may refer to a difference of methylation level value. For example, the methylation level difference may refer to an absolute value of the methylation level difference. For example, DMR regions with substantially consistent methylation levels can be determined by the methylation level differences between methylated sites and their adjacent methylated sites thereof. For example, the present application can make the classification of DMR regions more accurate through the sequencing coverage depth of a methylated site. For example, data information covering sites at higher depths is more reliable.

For example, the method may comprise determining an absolute value of methylation level difference between a methylated site and an adjacent methylated site thereof, and determining whether the methylated site and the adjacent methylated site thereof being classified into the same DMR based on the absolute value of the difference. For example, the method may comprise determining a weight for the absolute value of the difference, wherein the weight for the absolute value of the difference being determined according to the sequencing coverage depth of the methylated site. For example, the weight may be $e^{(1-P_{ij})}$, $P_{ij} \propto \dfrac{1}{e^{\frac{|d_{ij} - d_{i(j+1)}|}{d_{ij} + d_{i(j+1)}}}}$, wherein $d_{ij}$ is the sequencing coverage depth of the ith sample at the jth site.

For example, the method may comprise determining an absolute value of methylation level difference between a methylated site and an adjacent methylated site thereof, and determining a weight for the absolute value of the difference to determine a methylation level difference, wherein the weight for the absolute value of the difference being determined according to the sequencing coverage depth of the methylated site.

For example, the methylation level difference $\beta_{ij}$ is determined according to the following formula:

$$\beta_{ij} = \left| M_{ij} - M_{i\,(j+1)} \right| * e^{(1-P_{ij})}$$

wherein $M_{ij}$ is the methylation level of the ith sample at the jth site, e represents the natural constant, and $P_{ij}$ is determined according to the following formula:

$$P_{ij} \propto \frac{1}{e^{\frac{|d_{ij} - d_{i(j+1)}|}{d_{ij} + d_{i(j+1)}}}}$$

wherein $d_{ij}$ is the sequencing coverage depth of the ith sample at the jth site.

For example, the methylated site and the adjacent methylated site thereof are determined to be classified into the same DMR when the methylation level difference $\beta_{ij}$ of the methylated sites is less than or equal to about 0.25.

For example, the method may further comprise determining a degree of fluctuation of methylation level of the DMR, based on the difference in methylation levels of a methylated site inside the DMR and a methylated site at intermediate position of the DMR. For example, the intermediate position refers to an intermediate position in physical position. For example, when M is an odd number and the DMR has M methylated sites, the middle position may refer to the methylated site at about [(M+1)/2]th from upstream to downstream. For example, when M is an even number and the DMR has M methylated sites, the intermediate position may refer to the methylated site at about [M/2]th or at about [M/2+1]th from upstream to downstream.

For example, more preferable DMRs among the candidate DMRs are screened by judging the degree of fluctuation of methylation difference of each methylated site and methylation difference of a methylated site in the middle position in the candidate DMRs.

For example, the degree of fluctuation of methylation level of the DMR $B_{ij}$ is determined according to the following formula:

$$B_{ij} = \frac{1}{n} \sum_{j=1}^{n} \frac{|\beta_{ij} - \mu_j|}{\left|\beta_{ij} - \beta_{i\,(j+1)}\right|}$$

wherein $\beta_{ij}$ is the methylation level difference at the jth site of the ith sample, and $\mu_j$ is the methylation level difference of the methylated site at the intermediate position of DMR region. For example, determining a DMR with $B_{ij}$ less than about 1 being adopted for evaluating the correlation between the sample to be tested and the risk of tumor formation and/or tumor tissue of origin.

For example, the method may comprise evaluating the sample to be tested having risk of tumor formation through a binary classification model, based on the methylation level of DMR of the sample to be tested, wherein the method reduces influence of age factor of a subject on evaluation result of the correlation between the sample to be tested and the risk of tumor formation, wherein the sample to be tested is derived from the subject.

For example, the binary classification model may comprise a support vector machine SVM model. For example, the method may comprise introducing a penalty term based on the age factor into the SVM model. For example, the method may comprise introducing a penalty term based on the age factor in the SVM model by way of Hilbert-Schmidt independence criterion. For example, all the introduction way for penalty items that can be used for machine learning in the present application can be used for reducing the influence of age factor in the present application.

For example, the method may comprise preforming machine learning training for training samples known to have or to be free of tumor formation according to the following formula:

$$f(\mathrm{x}; \mathrm{w}, \mathrm{b}) = sgn\,(wT\mathrm{x} + b)$$

if a < 0, sgn (a) = -1 ; if a $\geq$ 0, sgn (a) = 1

and the following equation being adopted to determine training parameters:

$$\min_{w \in R^n, b \in R, \xi \in R^m} \frac{1}{2}\mathrm{w}T\mathrm{w} + C \sum_{i=1}^{m} \xi_i + \lambda L_H \left( P_{h(x)h(z)} \right)$$

s. t.

$$y_i(wTx + b) \geq 1 - \xi_i$$

$$\xi_i \geq 0$$

wherein $C$, w, $\lambda$, b represent training parameters, sgn() represents sign function, $\xi_i$ represents degree to which sample $x_i$ violates the equation, x represents methylation level of a sample, y represents as +1 when a sample being cancer tissue correlates with tumor formation, y represents as -1 when a sample being non-cancer tissue does not correlate with tumor formation, and $L_H(P_{h(x)h(z)})$ being determined by the following formula:

$$L_H\big(P_{h(x)h(z)}, F, G\big) := \big\|C_{h(x)h(z)}\big\|^2_{HS}$$

$$\big\|C_{h(y)h(z)}\big\|^2 = \big(E_{h(x)h(z)} - E_{h(x)}E_{h(z)}\big)^2 = \big(E_{h(x)h(z)}\big)^2 + \big(E_{h(x)}E_{h(z)}\big)^2 -$$

$$2E_{h(x)h(z)}E_{h(x)}E_{h(z)}$$

wherein $h(y)$ and $h(z)$ are kernel functions of $Y$ and $Z$ respectively, F and G represent the reproducing kernel Hilbert space of $X$ and Z respectively, $P_{h(x)h(z)}$ represents probability distribution of $h(y)$ and $h(z)$.

For example, the method may comprise evaluating the correlation between the sample to be tested and the tumor tissue of origin through determining classification probabilities by a multi-classification method, and fitting the classification probabilities by logistic regression, based on methylation level of the DMR of the sample to be tested. For example, the method determines classification probabilities by pairwise voting of binary classification. For example, the method may determine classification probabilities by various multi-classification methods in the art. For example, the method fits the classification probabilities by multiple linear regression MLR.

For example, the method may comprise performing regression analysis on training samples with known tissue of origin according to the following formula:

classification probabilities $p_i^v$ determined by binary classification being determined according to the following formula:

$$p_i^v = 2 \sum_{i:j \neq i} I_{\{r_{ij} > r_{ji}\}} / (k(k-1))$$

$$\mu_{ij} \equiv P(y = i \mid y = i \text{ or } j, x)$$

wherein I (x) is target equation: $I_{\{x\}}$ = 1 if x is true, $I_{\{x\}}$ = -1 if x is false, $r_{ij}$ is an estimate of pairwise classification probability $\mu_{ij}$, k is sum of tissue classes; i and j represent the ith and jth class respectively, and x represents methylation level of the DMR of a sample;

and weight $\beta_j$ for multiple linear regression MLR fitting being determined according to the following formula:

$$E\{Y_{ij}\} = \frac{exp\big(X'_i\beta_j\big)}{1 + \sum_{k=1}^{J-1} exp\big(X'_i\beta_k\big)} \quad j = 1, 2, \dots, J - 1$$

wherein $X'_i$ represents classification probabilities obtained by pairwise voting of binary classification, and $Y_{ij}$ represents tissue of origin class of a sample.

For example, the method corrects the tissue of origin of the training samples based on probability that the sample has tumor formation. For example, the method may comprise performing the correction before the pairwise voting of binary classification obtaining classification probabilities results. For example, the method may comprise performing the correction after the pairwise voting of binary classification obtaining classification probabilities results and before the multiple linear regression analysis. For example, the method may comprise performing the correction based on a quasi-maximum likelihood estimation method.

For example, the method may comprise performing the correction according to the following formula:

$$\prod_{i=1}^{n} f_i(y_i)^{w_i} = \prod_{i=1}^{n} \pi_i^{y_i w_i}(1 - \pi_i)^{(1-y_i)w_i}$$

wherein $y_i$ represents tissue of origin class of a sample, $w_i$ represents weight of the correction, $\pi_i$ represents probability that a sample has tumor formation. For example, through maximizing the expectation of this formula, the weights are determined such that the tissue of origin class can be corrected according to whether a sample has tumor formation. For example, information of the tissue of origin can be more reliable when evaluating the sample has tumor formation. In one aspect, the present application provides a system for evaluating correlation between a sample to be tested and risk of tumor formation and/or tumor tissue of origin, which may comprise: (1) a differentially methylated region DMR classification module: determining a plurality of target DMRs for evaluation, based on sequencing coverage depth of a methylated site and/or methylation level difference of adjacent methylated sites; (2) a tumor formation risk evaluation module: evaluating the correlation between the sample to be tested and the risk of tumor formation, based on methylation levels of the target DMRs of the sample to be tested; (3) optionally comprising a tumor tissue of origin evaluation module: evaluating the correlation between the sample to be tested and the tumor tissue of origin, based on methylation levels of the target DMRs of the sample to be tested.

In one aspect, the present application provides a system used for determining a differentially methylated region DMR, the system may comprise a differentially methylated region DMR classification module: determining a differentially methylated region DMR, based on sequencing coverage depth of a methylated site and/or methylation level difference of adjacent methylated sites.

In one aspect, the present application provides a system of evaluating correlation between a sample to be tested and risk of tumor formation, comprising a tumor formation risk evaluation module: evaluating the correlation between the sample to be tested and the risk of tumor formation, based on methylation level of the DMR of the sample to be tested, wherein the method comprising a module of reducing influence of age factor of a subject on evaluation result, wherein the sample to be tested is derived from the subject.

In one aspect, the present application provides a system of evaluating correlation between a sample to be tested and tumor tissue of origin, comprising a tumor tissue of origin evaluation module: evaluating the correlation between the sample to be tested and the tumor tissue of origin, based on methylation level of the DMR of the sample to be tested through a module of multi-classification method and logistic regression.

For example, the system may comprise determining the DMR based on sequencing coverage depth of a methylated site and the methylation level difference between a methylated site and an adjacent methylated site thereof. For example, the methylation level difference may refer to a difference of methylation level value. For example, the methylation level difference may refer to an absolute value of the methylation level difference. For example, DMR regions with substantially consistent methylation levels can be determined in the present application by the methylation level differences between methylated sites and their adjacent methylated sites thereof. For example, the present application can make the classification of DMR regions more accurate through the sequencing coverage depth of a methylated site. For example, data information covering sites at higher depths is more reliable.

For example, the system may comprise determining an absolute value of methylation level difference between a methylated site and an adjacent methylated site thereof, and determining whether the methylated site and the adjacent methylated site thereof being classified into the same DMR based on the absolute value of the difference. For example, the system may comprise determining a weight for the absolute value of the difference, wherein the weight for the absolute value of the difference being determined according to the sequencing coverage depth of the methylated site. For example, the weight may be $e^{(1-Pij)}$, $P_{ij} \propto \dfrac{1}{e^{\frac{|d_{ij} - d_{i(j+1)}|}{d_{ij} + d_{i(j+1)}}}}$, wherein $d_{ij}$ is the sequencing coverage depth of the ith sample at the jth site.

For example, the system may comprise determining an absolute value of methylation level difference between a methylated site and an adjacent methylated site thereof, and determining a weight for the absolute value of the difference to determine a methylation level difference, wherein the weight for the absolute value of the difference being determined according to the sequencing coverage depth of the methylated site.

For example, the methylation level difference $\beta_{ij}$ is determined according to the following formula:

$$\beta_{ij} = \left| M_{ij} - M_{i\,(j+1)} \right| * e^{(1-P_{ij})}$$

wherein, $M_{ij}$ is the methylation level of the ith sample at the jth site, e represents the natural constant, and $P_{ij}$ is determined according to the following formula:

$$P_{ij} \propto \frac{1}{e^{\frac{|d_{ij} - d_{i(j+1)}|}{d_{ij} + d_{i(j+1)}}}}$$

wherein $d_{ij}$ is the sequencing coverage depth of the ith sample at the jth site.

For example, the methylated site and the adjacent methylated site thereof are determined to be classified into the same DMR when the methylation level difference $\beta_{ij}$ of the methylated sites is less than or equal to about 0.25.

For example, the system may further comprise determining a degree of fluctuation of methylation level of the DMR, based on the difference in methylation level of a methylated site inside the DMR and a methylated site at intermediate position of the DMR. For example, the intermediate position refers to an intermediate position in physical position. For example, when M is an odd number and the DMR has M methylated sites, the middle position may refer to the methylated site at about [(M+1)/2]th from upstream to downstream. For example, when M is an even number and the DMR has M methylated sites, the intermediate position may refer to the methylated site at about [M/2]th or at about [M/2+1]th from upstream to downstream.

For example, more preferable DMRs among the candidate DMRs are screened by judging the degree of fluctuation of methylation difference of each methylated site and methylation difference of a methylated site in the middle position in the candidate DMRs.

For example, the degree of fluctuation of methylation level of the DMR $B_{ij}$ is determined according to the following formula:

$$B_{ij} = \frac{1}{n} \sum_{j=1}^{n} \frac{|\beta_{ij} - \mu_j|}{\left|\beta_{ij} - \beta_{i\ (j+1)}\right|}$$

wherein $\beta_{ij}$ is the methylation level difference at the jth site of the ith sample, and $\mu_j$ is the methylation level difference of the methylated site at the intermediate position of DMR region. For example, determining a DMR with $B_{ij}$ less than about 1 being adopted for evaluating the correlation between the sample to be tested and the risk of tumor formation and/or tumor tissue of origin.

For example, the system may comprise evaluating the sample to be tested having risk of tumor formation through a binary classification model, based on the methylation level of DMR of the sample to be tested, wherein the system reducing influence of age factor of a subject on evaluation result of the correlation between the sample to be tested and the risk of tumor formation and/or tumor tissue of origin, wherein the sample to be tested is derived from the subject.

For example, the binary classification model may comprise a support vector machine SVM model. For example, the system may comprise introducing a penalty term based on the age factor into the SVM model. For example, the system may comprise introducing a penalty term based on the age factor in the SVM model by way of Hilbert-Schmidt independence criterion. For example, all the introduction way for penalty items that can be used for machine learning in the present application can be used for reducing the influence of age factor in the present application.

For example, the system may comprise preforming machine learning training for training samples known to have or to be free of tumor formation according to the following formula:

$$f(\mathrm{x}; \mathrm{w}, \mathrm{b}) = sgn\ (wTx + b)$$

if a < 0, sgn (a) = -1 ; if a ≥ 0, sgn (a) = 1

and the following equation being adopted to determine training parameters:

$$\min_{w \in R^n, b \in R, \xi \in R^m} \frac{1}{2} \mathrm{wTw} + C \sum_{i=1}^{m} \xi_i + \lambda L_H \left( P_{h(x)h(z)} \right)$$

s. t.

$$y_i(wTx + b) \geq 1 - \xi_i$$

$$\xi_i \geq 0$$

wherein $C$, w, $\lambda$, b represent training parameters, sgn() represents sign function, $\xi_i$ represents degree to which sample $x_i$ violates the equation, x represents methylation level of a sample, y represents as +1 when a sample being cancer tissue correlates with tumor formation, y represents as -1 when a sample being non-cancer tissue does not correlate with tumor formation, and $L_H(P_{h(x)h(z)})$ being determined by the following formula:

$$L_H\left(P_{h(x)h(z)}, F, G\right) := \left\|C_{h(x)h(z)}\right\|_{HS}^2$$

$$\left\|C_{h(y)h(z)}\right\|^2 = \left(E_{h(x)h(z)} - E_{h(x)}E_{h(z)}\right)^2 = \left(E_{h(x)h(z)}\right)^2 + \left(E_{h(x)}E_{h(z)}\right)^2 - 2E_{h(x)h(z)}E_{h(x)}E_{h(z)}$$

wherein $h(y)$ and $h(z)$ are kernel functions of $Y$ and $Z$ respectively, F and G represent the reproducing kernel Hilbert space of $X$ and $Z$ respectively, and $P_{n(x)h(z)}$ represents probability distribution of $h(y)$ and $h(z)$.

For example, the system may comprise evaluating the correlation between the sample to be tested and the tumor tissue of origin through determining classification probabilities by a multi-classification method module, and fitting the classification probabilities by logistic regression, based on methylation level of the DMR of the sample to be tested. For example, the system determines classification probabilities by pairwise voting of binary classification. For example, the system may determine classification probabilities by various multi-classification method modules in the art. For example, the system fits the classification probabilities by multiple linear regression MLR.

For example, the system may comprise performing regression analysis on training samples with known tissue of origin according to the following formula:

classification probabilities $p_i^v$ determined by binary classification being determined according to the following formula:

$$p_i^v = 2 \sum_{i:j \neq i} I_{\{r_{ij} > r_{ji}\}} / (k(k-1))$$

$$\mu_{ij} \equiv P(y = i \mid y = i \text{ or } j, x)$$

wherein I (x) is target equation: $I_{\{x\}} = 1$ if $x$ is true, $I_{\{x\}} = -1$ if $x$ is false, $r_{ij}$ is an estimate of pairwise classification probability $\mu_{ij}$, k is sum of tissue classes; i and j represent the ith and jth class respectively, and $x$ represents methylation level of the DMR of a sample;

and weight $\beta_j$ for multiple linear regression MLR fitting being determined according to the following formula:

$$E\{Y_{ij}\} = \frac{exp\left(X'_i \beta_j\right)}{1 + \sum_{k=1}^{J-1} exp\left(X'_i \beta_k\right)} \quad j = 1, 2, \dots, J-1$$

wherein $X'_i$ represents classification probabilities obtained by pairwise voting of binary classification, and $Y_{ij}$ represents tissue of origin class of a sample.

For example, the system corrects the tissue of origin of the training samples based on probability that the sample has tumor formation. For example, the system may comprise performing the correction before the pairwise voting of binary classification obtaining classification probabilities results. For example, the system may comprise performing the correction after the pairwise voting of binary classification obtaining classification probabilities results and before the multiple linear regression analysis. For example, the system may comprise performing the correction based on a quasi-maximum likelihood estimation method module.

For example, the system may comprise performing the correction according to the following formula:

$$\prod_{i=1}^{n} f_i(y_i)^{w_i} = \prod_{i=1}^{n} \pi_i^{y_i w_i} (1 - \pi_i)^{(1-y_i)w_i}$$

wherein $y_i$ represents tissue of origin class of a sample, $w_i$ represents weight of the correction, and $\pi_i$ represents probability that a sample has tumor formation. For example, through maximizing the expectation of the equation, the weights are determined such that the tissue of origin class can be corrected according to whether a sample has tumor formation. For example, information of the tissue of origin can be more reliable when evaluating the sample has tumor formation.

In one aspect, the present application provides a storage medium, recording a program capable of operating the method described in the present application. For example, the non-volatile computer readable storage medium may include a floppy disk, a flexible disk, a hard disk, solid state storage (SSS) (e.g., solid state drive (SSD)), solid state card (SSC), solid state module (SSM), enterprise flash drive, magnetic tape, or any other non-transitory magnetic medium, etc. Non-volatile computer readable storage media may also include punch cards, paper tape, cursor sheets (or any other physical medium having a pattern of perforations or other optically identifiable indicia), compact disc read only memory (CD-ROM), compact disc rewritable (CD-RW), digital versatile disc (DVD), Blu-ray disc (BD), and/or any other non-transitory optical media.

In one aspect, the present application provides an apparatus, the apparatus comprising the storage medium as described in the present application, and the apparatus optionally comprising a processor coupled to the storage medium, and the processor being configured to implement the method as described in the present application, based on the program stored in the storage medium.

**Examples**

**Example 1**

Exemplary bisulfite-treated second-generation sequencing was performed on samples, resulting in sequencing data containing methylation levels and sequencing coverage depths for CpG at methylated sites. Optionally, noise removal was performed on genomic methylation signal CpG and CHH/CHG sites in noise region. Then, for the "tumor"(C) and "normal"(N) groups, the p-values obtained by weighted logistic regression were calculated. The explanatory variables of logistic regression were adopted as continuous variables, i.e., methylation levels at each CpG point, and the response variables were adopted as binary output, i.e., (0, 1), corresponding to C and N. Weighted logistic regression did the test to distinguish C from N at each CpG site. The null hypothesis is that the difference between C and N at that CpG site is not statistically significant. Weights are determined based on the depth of coverage of each CpG site.

DMR classification

Determine how DMR regions are divided based on methylation levels and sequencing coverage depths of CpG at methylated sites. Specifically, the methylation level and sequencing coverage depth of CpG at methylated sites are calculated according to the following formula:

$$P_{ij} \propto \frac{1}{e^{\frac{|d_{ij} - d_{i\,(j+1)}|}{d_{ij} + d_{i\,(j+1)}}}}$$

$$\beta_{ij} = \left| M_{ij} - M_{i\,(j+1)} \right| * e^{(1-P_{ij})}$$

$$\beta_{ij} \begin{cases} < 0.25, & \text{the } j\text{th and } (j+1)\text{th sites can be substituted into the calculated region} \\ & \text{statistic B and possibly be classified into a DMR} \\ \geq 0.25, & \text{the } j\text{th and } (j+1)\text{th sites can not be substituted into the calculated region} \\ & \text{statistic B and will not be classified into a DMR} \end{cases}$$

**EP 4 567 817 A1**

The $d_{ij}$ herein is the effective coverage depth of the jth site in the ith sample of group C, and the $M_{ij}$ is the methylation level of the jth site in the ith sample of group C. The methylation level similarity of consecutive sites in genome space was evaluated. The deeper the coverage depth, the larger the value of parameter P, the higher the methylation level approximation between adjacent CpG sites within the same group.

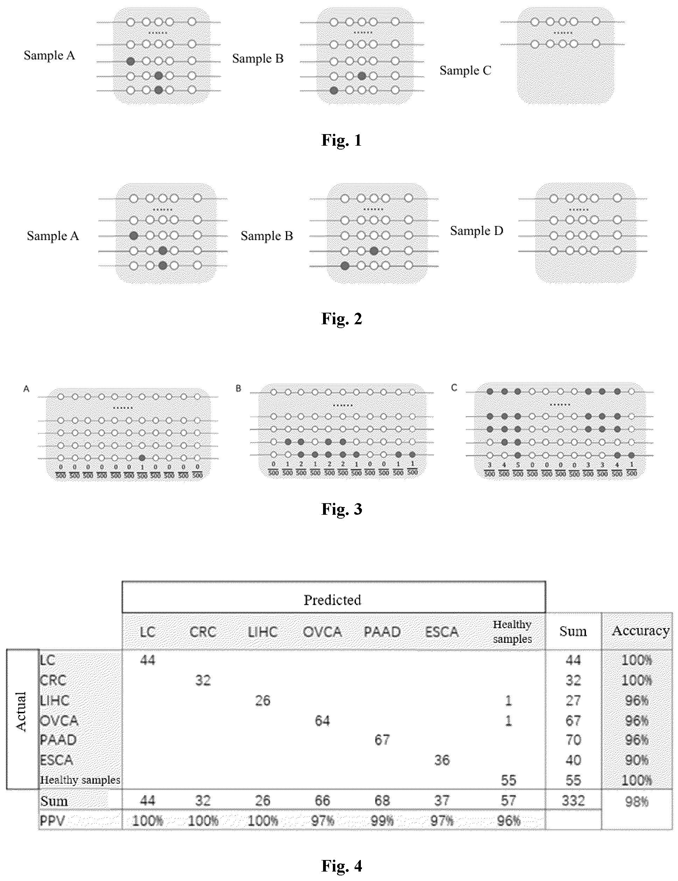

Fig. 1 shows an exemplary scenario (a theoretical exemplary demonstration, not intended to represent actual sequencing situation).

For the first CpG site in the region, sample A and sample B obtained coverage of 500 valid sequences respectively, and sample C obtained coverage of 200 valid sequences. For sample A, the methylation level of this CpG site was 0.2. The methylation level of the second CpG site in sample A was 0. The coverage depth parameter P of the first CpG site for three samples in the group was calculated and was 0.617. At this moment, $\beta_{ij} = |0.2 - 0| * e^{(1-0.617)} = 0.29$. Since the methylation difference between two CpG sites before and after was less than 0.25 being one of the necessary conditions for classifying the two adjacent loci into the same DMR, the first and second CpG sites in this example will not be classified into the same DMR.

Fig. 2 shows another exemplary scenario (a theoretical exemplary demonstration, not intended to represent actual sequencing situation).

If the above samples are replaced by A, B, D (wherein sample D obtained coverage of 400 valid sequences at the first CpG site). Similarly, for sample A, the methylation level of this CpG site is 0.2. The methylation level of the second CpG site in sample A was 0. However, due to the increased sequencing coverage of sample D in this example, the coverage depth parameter P of the first CpG site for three samples in the group was calculated to be 0.962. At this moment, $\beta_{ij} = |0.2 - 0| * e^{(1-0.962)} = 0.21$, and was less than 0.25 which is the threshold for being classified into the same DMR, thus the first and second CpG sites in this example of sample A meet the prerequisite of being classified into the same DMR.

Therefore, by introducing the coverage depth of CpG sites through the method of the present application, the accuracy of DMR region division can be significantly improved.

Further optionally, for $B_{ij}$ within a region, the method of calculation is as follows

$$B_{ij} = \frac{1}{n}\sum_{j=1}^{n}\frac{|\beta_{ij} - \mu_j|}{\left|\beta_{ij} - \beta_{i\,(j+1)}\right|}$$

$$B_{ij}\begin{cases}< 1, & \text{can be regarded as valid } DMR \\ \geq 1, & \text{not be regarded as valid } DMR\end{cases}$$

Figs.3A-3C show another exemplary scenario (a theoretical exemplary demonstration, not intended to represent actual sequencing situation). When the DMR region contains 10 CpG sites, the scores for each DMR were calculated by combining $B_{ij}$ of all samples together and by averaging.

The calculation steps for B values in DMR region indicated by group A are shown in the following table:

| Site No. $j$ | 1 | 2 | 3 | 4 | 5 | 6 | 7 | 8 | 9 | 10 | 11 |
|---|---|---|---|---|---|---|---|---|---|---|---|
| $\beta_{ij}$ | $\frac{0}{500}$ | $\frac{0}{500}$ | $\frac{0}{500}$ | $\frac{0}{500}$ | $\frac{0}{500}$ | $\frac{0}{500}$ | $\frac{1}{500}$ | $\frac{0}{500}$ | $\frac{0}{500}$ | $\frac{0}{500}$ | $\frac{0}{500}$ |
| $\|\beta_{ij} - \mu_j\|$ | $\frac{0}{500}$ | $\frac{0}{500}$ | $\frac{0}{500}$ | $\frac{0}{500}$ | $\frac{0}{500}$ | $\frac{0}{500}$ | $\frac{1}{500}$ | $\frac{0}{500}$ | $\frac{0}{500}$ | $\frac{0}{500}$ | / |
| $\beta_{i\,(j+1)}$ | $\frac{0}{500}$ | $\frac{0}{500}$ | $\frac{0}{500}$ | $\frac{0}{500}$ | $\frac{0}{500}$ | $\frac{1}{500}$ | $\frac{0}{500}$ | $\frac{0}{500}$ | $\frac{0}{500}$ | $\frac{0}{500}$ | / |
| $\|\beta_{ij} - \beta_{i\,(j+1)}\|$ | $\frac{0}{500}$ | $\frac{0}{500}$ | $\frac{0}{500}$ | $\frac{0}{500}$ | $\frac{0}{500}$ | $\frac{1}{500}$ | $\frac{1}{500}$ | $\frac{0}{500}$ | $\frac{0}{500}$ | $\frac{0}{500}$ | / |
| $\frac{\|\beta_{ij}-\mu_j\|}{\left\|\beta_{ij}-\beta_{i\,(j+1)}\right\|}$ | / | / | / | / | / | 0 | 1 | / | / | / | / |

The B value was scored to be 0.1, i.e., $\frac{1}{10} \times (0 + 1) = 0.1$.

Similarly, the B value within the DMR shown in group B was scored to be 0.7, i.e., $\frac{1}{10} \times (2 + 1+0+1+0+1+2)$, and the B values within the DMR shown in group C was scored to be 1.233, i.e., $\frac{1}{10} \times (3 + 4 + 1 + 0 + 3 + 1.33)$.

DMR regions screened by this method not only contain cancer mutation information of various cancer types, but also contain tissue-specific features, and have better segmentation effects at region boundaries.

Fig. 4 shows that accuracy of 98% (95% CI: 96-99%) for tissue of origin can be achieved in 5-fold cross-validation for 6 types of cancers: lung carcinoma (LC, Lung Carcinoma), colorectal carcinoma (CRC, Colorectal Carcinoma), liver hepatocellular carcinoma (LIHC, Liver Hepatocellular Carcinoma), ovarian carcinoma (OVCA, Ovarian Carcinoma), pancreatic adenocarcinoma (PAAD, Pancreatic Adenocarcinoma), and esophageal carcinoma (ESCA, Esophageal Carcinoma).

**Example 2**

Detection Of Cancer (DOC) Modeling

The ctDNA content in blood varies greatly at different stages of cancer development and is susceptible to experimental batch effects. In addition, methylation variants are associated with age, disease, race, etc., which if left untreated, may affect the accuracy of classification models as confounding variables. The present application adopts a model construction method called Salmon, which firstly quantifies the bias brought by confounding variables (the quantization method can adopt but is not limited to Hilbert-Schmidt independence criterion), and then performed corrections with embedding into the regularization in the model, to increase the accuracy and generalization ability of the model.

Algorithm Establishment

Assuming $m$ samples, set feature vector $X$ $(x_1, \dots , x_m)$, classification label $Y$ $(y_1, \dots , y_m)$, confounding variable $Z(z_1, \dots , z_m)$, where $x_i$ is a $n$ dimensional vector representing the methylation feature of the sample $i$, $y_i$ is classification label of the sample $x_i$, $y_i \in \{-1, +1\}$, $z_i$ is some confounding variable of the sample $i$.

$$L_H \left( P_{h(x)h(z)}, F, G \right) := \left\| C_{h(x)h(z)} \right\|^2_{HS}$$

The $L_H$ herein refers to Hilbert-Schmidt independence criterion, which is used to measure the degree of independence of variables $X$ and $Z$, $h(y)$ and $h(z)$ are kernel functions of $Y$ and $Z$, $P_{h(x)h(z)}$ represents the probability distribution of $h(y)$ and $h(z)$, F and G represent the reproducing kernel Hilbert space of $X$ and $Z$ respectively, which can be understood as the domain mapped after nonlinear processing of $X$ and $Z$, $C_{h(x)h(z)}$ refers to the correlation coefficient of these two kernel functions, and HS is Hilbert Space.

$$\left\| C_{h(y)h(z)} \right\|^2 = \left( E_{h(x)h(z)} - E_{h(x)}E_{h(z)} \right)^2$$

$$= \left( E_{h(x)h(z)} \right)^2 + \left( E_{h(x)}E_{h(z)} \right)^2 - 2 E_{h(x)h(z)} E_{h(x)}E_{h(z)}$$

Support vector machine (SVM) is adopted as main classifier

$$f(\mathrm{x}; \mathrm{w}, \mathrm{b}) = sgn \left( wT\mathrm{x} + b \right)$$

$$sgn \left( a \right) = 1(-1) \; if \; a \geq 0(< 0)$$

The classification interface is determined by solving the following target equation,

$$\min_{w \in R^n, b \in R} \frac{1}{2} \mathrm{wTw}$$

s.t.

$$y_i(wT\mathrm{x} + b) \geq 1$$

For non-separable data, soft-margin SVM is introduced with a penalty term for training errors

$$\min_{w \in R^n, b \in R, \xi \in R^m} \frac{1}{2} \mathrm{wTw} + C \sum_{i=1}^{m} \xi_i$$

s.t.

$$y_i(wT\mathrm{x} + b) \geq 1 - \xi_i$$

$$\xi_i \geq 0$$

Herein the balance of minimizing training error and maximizing classification margin is controlled by $C$, and $\xi_i$ refers to the degree to which the sample $x_i$ violates the equation.

In order to control the confounding factors, Salmon adds a regularization into the objective equation solved by SVM, and the parameter $\lambda$ controls the balance between the confounding factor error and the maximizing boundary width in the training, the objective equation is

$$\min_{w \in R^n, b \in R, \xi \in R^m} \frac{1}{2} \mathrm{wTw} + C \sum_{i=1}^{m} \xi_i + \lambda L_H \left( P_{h(x)h(z)} \right)$$

s.t.

$$y_i(wT\mathrm{x} + b) \geq 1 - \xi_i$$

$$\xi_i \geq 0$$

The balance among minimizing training errors, minimizing correlations between confounding variables and explanatory variables, and maximizing classification intervals is controlled by C and $\lambda$ herein.

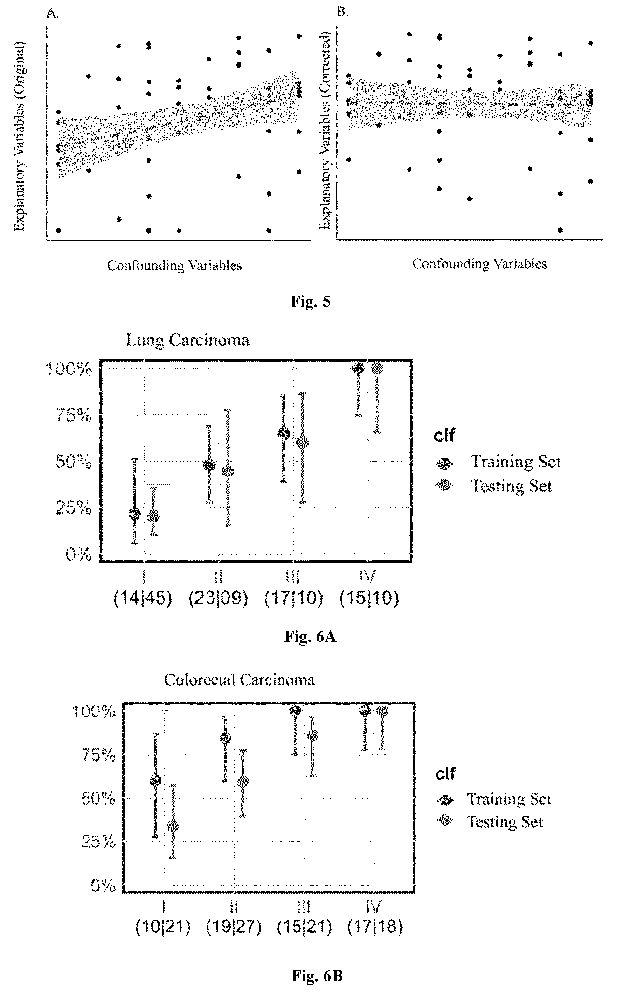

Fig. 5 shows the results of controlling the weight allocation of confounding related features in the Salmon-DOC model of the present application.

Each data point represents a blood sample used in the Salmon-DOC model construction, with the horizontal axis showing the corresponding sample's confunding factor and the vertical axis showing the original uncorrected variable coef (Figure A) and the corrected variable coef (Figure B). Comparation between the before and after correction shows that the weights of confounding correlation features are controlled in Salmon-DOC.

Data of Retrospective Cohort

The present application adopts retrospective clinical samples of 6 cancer types, which are divided into Training set and Validation set to evaluate the accuracy of Salmon binary classifier (cancer vs non-cancer).

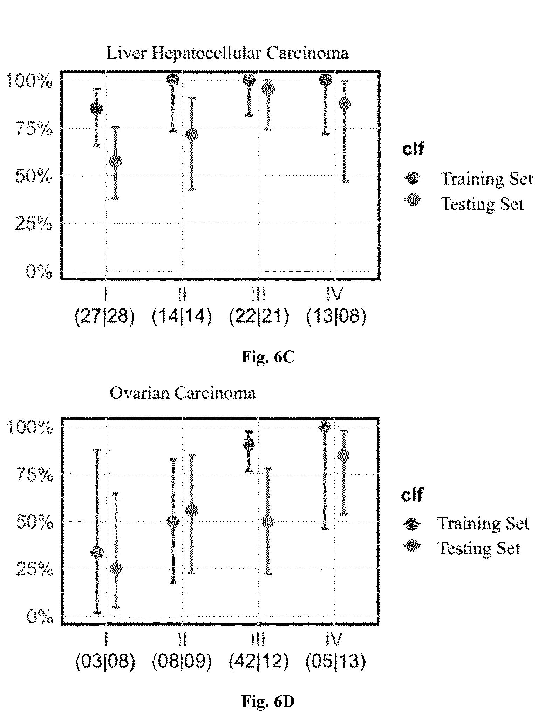

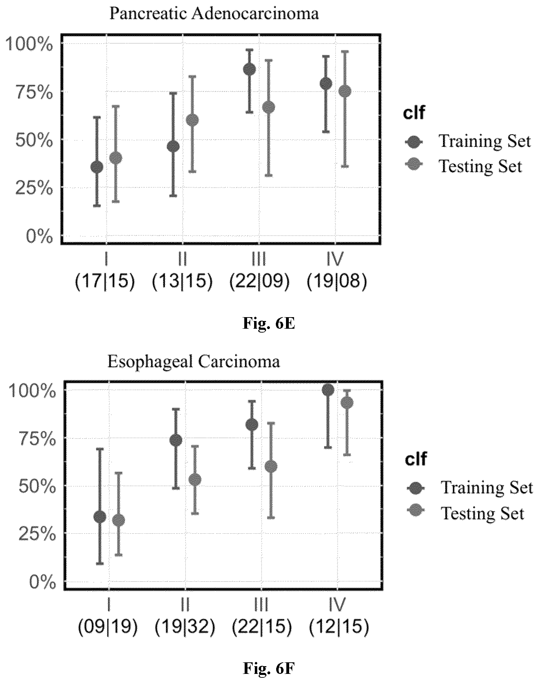

Figs.6A-6F show that the Salmon-DOC model of the present application can efficiently detect 6 cancer types at different stages in the tumor group model.

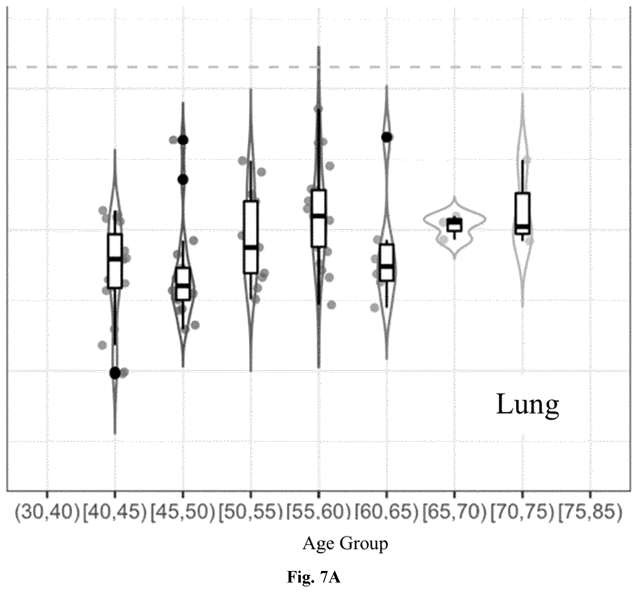

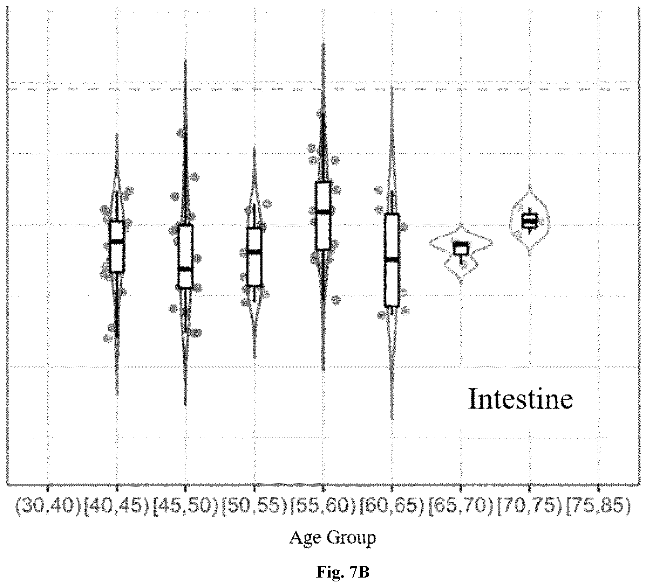

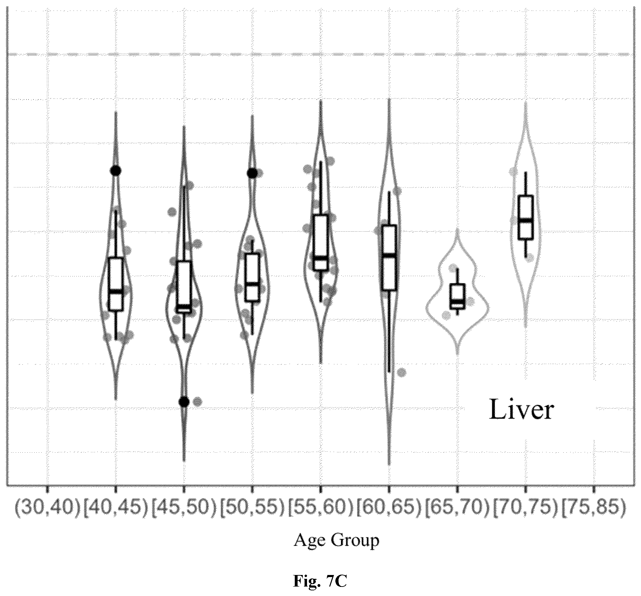

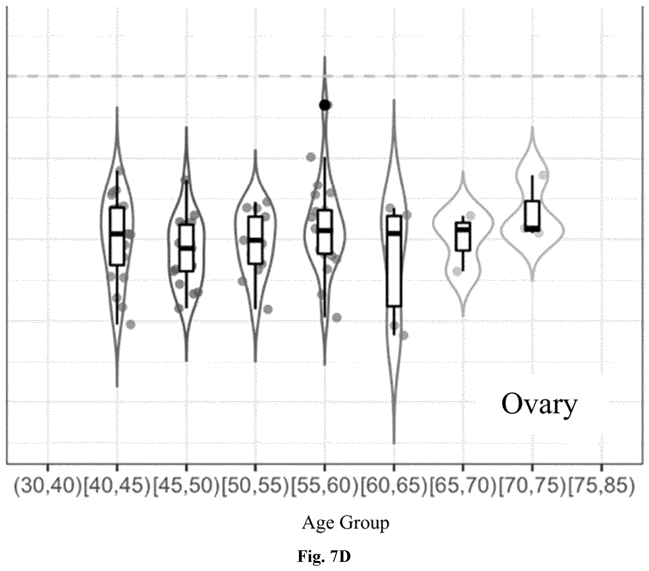

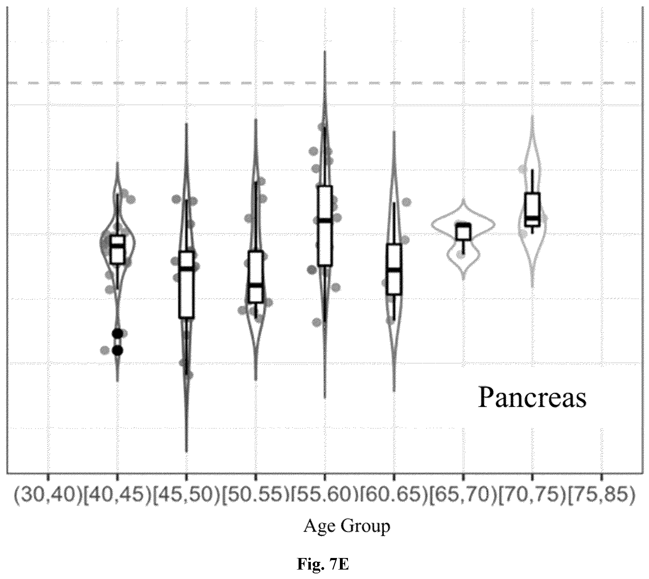

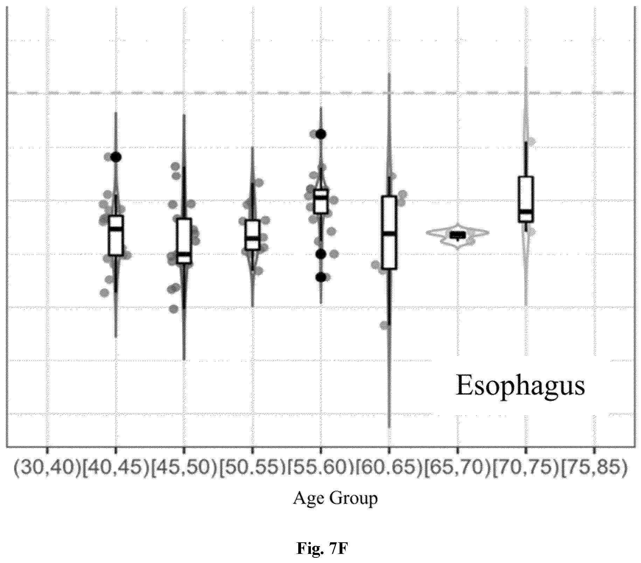

Figs.7A-7F show that the Salmon-DOC model of the present application overcomes the weakness of the previous that false positives of methylation increasing with age in the healthy group and remains balanced across each age groups (age on the horizontal axis and cancer probability scores on the vertical axis).

**Example 3**

Establishment of Tissue Of Origin (TOO) Model

Construction of the first layer of TOO model

The TOO model is essentially a multi-classification problem. The probability calculation for each class can be simplified to voting on pairs of pairwise results and then selecting the result with the most votes. For possible clinical applications of Tissue Of Origin (TOO) Model, however, it is not sufficient to produce only one classification result; only the probability of classification produced can make assembly of the model possible.

Therefore, the first step of the Salmon-TOO model of the present application is to quantify the voting results of the binary classification. This quantification can be proved by probability calculations. If some data point x and label y are defined, we assume that pairwise classification probabilities $\mu_{ij}$ exists, then from the ith and jth classes in the training set, we can obtain a model that can adopt the $r_{ij}$ calculated as the be approximate estimate of $\mu_{ij}$ as long as any new data point x is input. The problem can be simplified to be estimating the probability of the ith class using all $r_{ij}$

$$p_i = P(y = i \mid x), i = 1, \dots, k$$

define $r_{ij}$ as the estimation of $\mu_{ij}$, assuming $\mu_{ij} + \mu_{ji} = 1$. For multi-classification problems, the voting system is adopted,

$$\mu_{ij} \equiv P(y = i \mid y = i \text{ or } j, x)$$

$$\delta V = arg\ \max_i \left[ \sum_{i : j \neq i} I_{\{r_{ij} > r_{ji}\}} \right]$$

Define I is the target equation: $I_{\{x\}} = 1$ if *x* is true, otherwise being false. Probability calculation can be written as

$$p_i^v = 2 \sum_{i : j \neq i} I_{\{r_{ij} > r_{ji}\}} / (k(k-1))$$

Construction of the second layer of TOO model

The second level of the Salmon-TOO model is conducting MLR fitting for different classes.

Assuming that probability calculation needs to be performed on the tissue source, the quantized binary classification probability can be obtained according to the first layer, and the value range is $(\infty, -\infty)$. Because the actual distribution of each pair of binary probabilities is inconsistent, the quantized binary probabilities can be further used as explanatory variables of logistic regression, and the response variables adopt multivariate outputs corresponding to the known tissue sources in the modeling process.

| | $x_1$ | $x_2$ | $x_3$ | | $x_{C_2^J}$ |
|---|---|---|---|---|---|
| | (*class*$_1$ vs. *class*$_2$) | (*class*$_1$ vs. *class*$_3$) | ( *class*$_1$ vs. *class*$_4$) | ...... | (*class*$_{J-1}$ vs. *class*$_J$) |
| $y_1$ (*class*$_1$) | 3.0 | 6.1 | 5.2 | ...... | 0.1 |
| $y_2$ (*class*$_2$) | -1.5 | 0.2 | -0.1 | ...... | 0.0 |
| $y_3$ (*class*$_3$) | 0.1 | 7.2 | -0.1 | ...... | -0.8 |
| ...... | ...... | ...... | ...... | ...... | ...... |
| $y_J$ (*class*$_J$) | 0.4 | -0.7 | 0.1 | ...... | -9.0 |

As shown in the table above, each column represents a characteristic variable $x_{C_2^J}$ of logistic regression, i.e., the probability of bi-categorical evaluation of each two tissue classes; each row represents a response variable $y_1$, i.e., tissue class.

For the characteristic variables used to explain the probability of binomial classification, assuming that there are *J* discontinuous reflection variables in total, and the evaluation results are transformed into $Y_{i1}, \cdots, Y_{iJ}$, $\beta_j$ is the characteristic weights based on each reflection variable.

$$E\{Y_{ij}\} = \frac{exp\left(X'_i\beta_j\right)}{1 + \sum_{k=1}^{J-1} exp\left(X'_i\beta_k\right)} \quad j = 1, 2, \ldots, J-1$$

Since in the Salmon-DOC model, we can obtain that it is judged negative in some cancer types and positive in some cancer types, so for this judgment, when performing the Origin modeling, the weight correction based on the quasi-maximum likelihood estimation method is performed on the tissue class. Taking binary logistic regression as an example, it can be interpreted as:

$$\prod_{i=1}^{n} f_i(y_i)^{w_i} = \prod_{i=1}^{n} \pi_i^{y_i w_i}(1 - \pi_i)^{(1-y_i)w_i}$$

Data of Retrospective Cohort

All data from the retrospective cohort were 1:1 split randomly into a Training set and a Validation set. First, the origin evaluation results are obtained by cross-validation of the Training set, and the model parameters are continuously optimized in the process and finally locked. Finally, all data from the Validation set were evaluated for origin by the locked model. In the Training set of the Origin model, the sample size of six cancer types is 300 in total, and the number of each cancer type and stage is relatively balanced: 36 cases of lung carcinoma (4/12/5/15 cases in stages I~IV), 62 cases of colorectal carcinoma (8/18/18/18 cases in stages I~IV), 74 cases of liver hepatocellular carcinoma (25/14/22/13 cases in stages I~IV), 48 cases of ovarian carcinoma (1/4/38/5 cases in stages I~IV), 40 cases of pancreatic adenocarcinoma (3/6/13/18 cases in stages I~IV), 42 cases of esophageal carcinoma (5/10/15/12 cases in stages I~IV). There are 224 samples in the Validation set of the Origin model, including: 31 cases of lung carcinoma (4/5/12/10 cases in stages I~IV); 52 cases of colorectal carcinoma (7/15/13/17 cases in stages I~IV), 55 cases of hepatocellular carcinoma (17/11/20/7 cases in stages I~IV), 27 cases of ovarian carcinoma (3/4/8/12 cases in stages I~IV), 25 cases of pancreatic adenocarcinoma (4/6/6/9 cases in stages I~IV) and 34 cases of esophageal carcinoma (4/7/8/15 cases in stages I~IV).

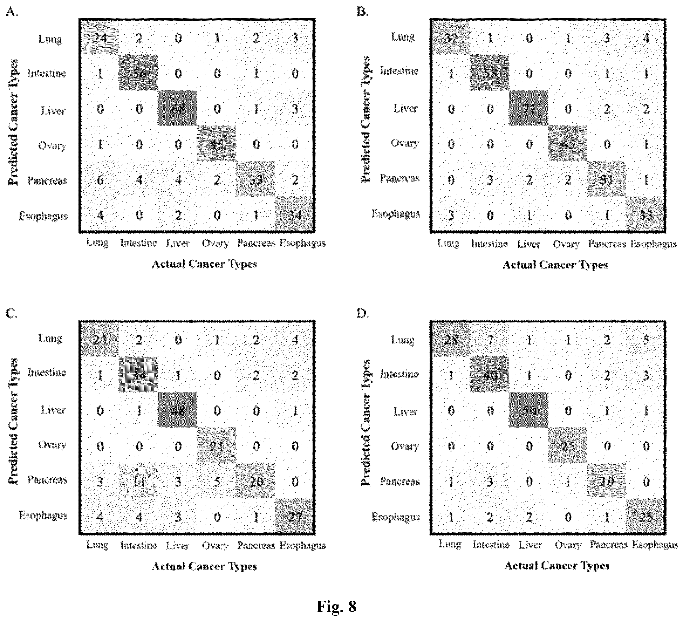

Figs. 8A-8D show that the accuracy of origin of the Salmon-TOO two-layer model of the present application is better than that of the single-layer model in both cross-validation and independent validation.

Figs. 8A and 8B show the origin evaluation results of cross-validation of the data of six cancer types in the six-cancer-type Training set. Among them, Fig. 8A shows the output results after only the first TOO model was constructed, and the accuracy of origin was 0.87 (260/300). If the suboptimal origin results are included, the accuracy was 0.93 (279/300); Fig. 8B shows the output results after supplementing the second-level MLR model on the basis of the first-level TOO model with the accuracy of origin improved to 0.90 (270/300), and the accuracy can be further improved to 0.95 (284/300) if the sub-optimal origin results was included. Similarly, Figs. 8C and 8D show the origin evaluation results of independent verification of the data of six cancer types in the above Validation set. Among them, Fig. 8C shows the output results only the first TOO model was constructed, and the accuracy of origin was 0.77 (173/224). If the suboptimal origin results are included, the accuracy was 0.87 (194/224); Fig. 8D shows the output results after supplementing the second-level MLR model on the basis of the first-level TOO model with the accuracy of origin improved to 0.84 (187/224), and the accuracy can be further improved to 0.89 (199/224) if the sub-optimal origin results was included.

In summary, the Salmon-TOO two-layer Origin model of the present application demonstrates better evaluation accuracy than the single-layer model in both Training set cross-validation and independent validation.

**Example 4**

DOC Detection Of Cancer Model

Table 1A shows the 94 DMR regions adpoted in the DOC cancer detection model

| Chromosome Numbering | Starting Coordinate | Ending Point Coordinate | Chromosome Numbering | Starting Coordinate | Ending Point Coordinate | Chromosome Numbering | Starting Coordinate | Ending Point Coordinate |
|---|---|---|---|---|---|---|---|---|
| 1 | 12655194 | 12655421 | 6 | 10884092 | 10884316 | 14 | 50527870 | 50528014 |
| 1 | 36042931 | 36043301 | 6 | 24358259 | 24358357 | 14 | 85998437 | 85998850 |
| 1 | 57889542 | 57889783 | 6 | 42072089 | 42072326 | 15 | 37170394 | 37170556 |
| 1 | 66258710 | 66258919 | 6 | 43252804 | 43253017 | 15 | 41787759 | 41787950 |
| 1 | 94702665 | 94702906 | 6 | 74290168 | 74290371 | 15 | 64456031 | 64456090 |
| 1 | 111217138 | 111217787 | 6 | 136358883 | 136359048 | 15 | 65134265 | 65134456 |
| 1 | 198651112 | 198651193 | 7 | 86273683 | 86273892 | 15 | 73989524 | 73989576 |
| 1 | 198904137 | 198904342 | 7 | 87848665 | 87848853 | 16 | 103520 | 103731 |
| 1 | 226867905 | 226868009 | 7 | 99517220 | 99517409 | 16 | 29819033 | 29819240 |
| 1 | 228645426 | 228645690 | 8 | 82192468 | 82192699 | 16 | 29887965 | 29888205 |
| 1 | 243646393 | 243646583 | 8 | 110986318 | 110987163 | 16 | 54970257 | 54970478 |
| 2 | 1747021 | 1748795 | 8 | 121822423 | 121822624 | 16 | 86527879 | 86528007 |
| 2 | 29337908 | 29338440 | 9 | 71788714 | 71788912 | 16 | 88496952 | 88497148 |
| 2 | 30371367 | 30371514 | 9 | 71789479 | 71789804 | 17 | 29298094 | 29298735 |
| 2 | 71192000 | 71192568 | 9 | 100747421 | 100747647 | 17 | 33390708 | 33390856 |
| 2 | 85804601 | 85804852 | 10 | 65029113 | 65029339 | 17 | 46655103 | 46656182 |
| 2 | 85811380 | 85811605 | 10 | 93999632 | 93999840 | 17 | 72348118 | 72348323 |
| 2 | 176931652 | 176933468 | 10 | 94821612 | 94821821 | 17 | 75369454 | 75369665 |
| 2 | 208492002 | 208492183 | 10 | 103875120 | 103875157 | 17 | 75370282 | 75370463 |
| 2 | 219735814 | 219736484 | 10 | 104433954 | 104434182 | 18 | 63417383 | 63417847 |
| 2 | 238583502 | 238583678 | 10 | 116286372 | 116286976 | 19 | 13210296 | 13210526 |
| 3 | 96532772 | 96533661 | 10 | 134121362 | 134121537 | 19 | 23299837 | 23300068 |
| 3 | 147129890 | 147130046 | 11 | 64993204 | 64993419 | 19 | 39360409 | 39360643 |
| 3 | 169382104 | 169382265 | 11 | 113929754 | 113929967 | 19 | 45901589 | 45901813 |
| 3 | 192125963 | 192126200 | 12 | 14134428 | 14134635 | 19 | 49127279 | 49127709 |
| 4 | 39045787 | 39045942 | 12 | 25055909 | 25056424 | 19 | 56904839 | 56905081 |
| 4 | 42153636 | 42153877 | 12 | 30975737 | 30976018 | 20 | 58514747 | 58514974 |
| 4 | 170947181 | 170947417 | 12 | 53108103 | 53108332 | 21 | 26934381 | 26934887 |
| 5 | 74965037 | 74965188 | 12 | 111842870 | 111843076 | 22 | 17082523 | 17082761 |
| 5 | 139047953 | 139048324 | 12 | 121570748 | 121570895 | 22 | 24236256 | 24236465 |
| 5 | 140797161 | 140797369 | 12 | 122519672 | 122519821 | | | |
| 5 | 142781721 | 142781863 | 14 | 30396461 | 30396694 | | | |

Based on 94 DOC-related DMR regions, 100 healthy human samples and 318 six-cancer-positive samples in independent Validation set 1 were evaluated with an overall sensitivity of 80.5%(256/318) and an overall specificity of 95% (95/100). While maintaining specificity at 90%, the sensitivities for specific cancer types and stages are as shown in the Table below:

| Cancer Types | Stages | Sample Size | Number of Positive Cases | Sensibility |
|---|---|---|---|---|
| Lung Carcinoma | Sum | 71 | 50 | 70.4% |
| | Stage I | 10 | 2 | 20.0% |
| | Stage II | 4 | 1 | 25.0% |
| | Stage III | 28 | 20 | 71.4% |
| | stage IV | 29 | 27 | 93.1% |
| Colorectal Carcinoma | Sum | 40 | 33 | 82.5% |
| | Stage I | 8 | 6 | 75.0% |
| | Stage II | 9 | 7 | 77.8% |
| | Stage III | 15 | 12 | 80.0% |
| | stage IV | 8 | 8 | 100.0% |
| Hepatocellular Carcinoma | Sum | 66 | 62 | 93.9% |
| | Stage I | 15 | 12 | 80.0% |
| | Stage II | 16 | 15 | 93.8% |
| | Stage III | 18 | 18 | 100.0% |
| | stage IV | 17 | 17 | 100.0% |
| Ovarian Carcinoma | Sum | 58 | 49 | 84.5% |
| | Stage I | 3 | 1 | 33.3% |
| | Stage II | 6 | 3 | 50.0% |
| | Stage III | 24 | 20 | 83.3% |
| | stage IV | 25 | 25 | 100.0% |
| Pancreatic Adenocarcinoma | Sum | 53 | 39 | 73.6% |
| | Stage I | 13 | 4 | 30.8% |
| | Stage II | 13 | 8 | 61.5% |
| | Stage III | 9 | 9 | 100.0% |
| | stage IV | 18 | 18 | 100.0% |
| Esophageal Carcinoma | Sum | 30 | 23 | 76.7% |
| | Stage I | 4 | 1 | 25.0% |
| | Stage II | 9 | 5 | 55.6% |
| | Stage III | 8 | 8 | 100.0% |
| | stage IV | 9 | 9 | 100.0% |

Repeated tests were then conducted, for each adopting random 50 out of 94 DOC regions. The sensitivity results of the six-cancer-positive samples in five replicates while maintaining specificity at the 90%(90/100) level are shown in the Table below:

| Cancer Types | Total number of Cases | Replicate 1 | | Replicate 2 | | Replicate 3 | | Replicate 4 | | Replicate 5 | |
|---|---|---|---|---|---|---|---|---|---|---|---|
| | | Number of positive cases | Sensibility | Number of positive cases | Sensibility | Number of positive cases | Sensibility | Number of positive cases | Sensibility | Number of positive cases | Sensibility |
| Lung Carcinoma | 71 | 44 | 62.0% | 45 | 63.4% | 48 | 67.6% | 44 | 62.0% | 47 | 66.2% |
| Colorectal Carcinoma | 40 | 31 | 77.5% | 29 | 72.5% | 29 | 72.5% | 32 | 80.0% | 32 | 80.0% |

(continued)

| Cancer Types | Total number of Cases | Replicate 1 | | Replicate 2 | | Replicate 3 | | Replicate 4 | | Replicate 5 | |
|---|---|---|---|---|---|---|---|---|---|---|---|
| | | Number of positive cases | Sensibility | Number of positive cases | Sensibility | Number of positive cases | Sensibility | Number of positive cases | Sensibility | Number of positive cases | Sensibility |
| Hepatocellular Carcinoma | 66 | 59 | 89.4% | 60 | 90.9% | 58 | 87.9% | 59 | 89.4% | 60 | 90.9% |
| Ovarian Carcinoma | 58 | 45 | 77.6% | 44 | 75.9% | 46 | 79.3% | 47 | 81.0% | 45 | 77.6% |
| Pancreatic Adenocarcinoma | 53 | 33 | 62.3% | 37 | 69.8% | 38 | 71.7% | 38 | 71.7% | 35 | 66.0% |
| Esophageal Carcinoma | 30 | 20 | 66.7% | 22 | 73.3% | 21 | 70.0% | 22 | 73.3% | 22 | 73.3% |

## Example 5

TOO Tissue Of Origin Model

Table 1B shows the 103 DMR regions adopted for the TOO Tissue Of Origin Model

| Chromosome Numbering | Starting Coordinate | Ending Point Coordinate | Chromosome Numbering | Starting Coordinate | Ending Point Coordinate | Chromosome Numbering | Starting Coordinate | Ending Point Coordinate |
|---|---|---|---|---|---|---|---|---|
| 1 | 16482493 | 16482728 | 5 | 139047953 | 139048324 | 13 | 28502583 | 28503595 |
| 1 | 111217138 | 111217787 | 5 | 139227564 | 139227787 | 13 | 61987902 | 61988077 |
| 1 | 198651112 | 198651193 | 5 | 167956245 | 167956565 | 14 | 105714537 | 105715205 |
| 1 | 198904137 | 198904342 | 6 | 24358259 | 24358357 | 15 | 45479588 | 45479962 |
| 1 | 206680198 | 206680422 | 6 | 42072089 | 42072326 | 15 | 73989524 | 73989576 |
| 1 | 214156143 | 214156385 | 6 | 74290168 | 74290371 | 15 | 101513738 | 101513975 |
| 1 | 226867905 | 226868009 | 6 | 78173672 | 78173887 | 16 | 103520 | 103731 |
| 1 | 226925137 | 226925338 | 6 | 84418729 | 84418959 | 16 | 22019277 | 22019517 |
| 1 | 228194384 | 228194584 | 7 | 3341470 | 3341704 | 16 | 54970257 | 54970478 |
| 1 | 240255077 | 240255308 | 7 | 4923086 | 4923319 | 17 | 6659010 | 6659487 |
| 1 | 243646393 | 243646583 | 7 | 37487553 | 37487794 | 17 | 29298094 | 29298735 |
| 2 | 29337908 | 29338440 | 7 | 99517220 | 99517409 | 17 | 38347541 | 38347979 |
| 2 | 54786104 | 54786165 | 8 | 37655799 | 37656022 | 17 | 38478748 | 38478962 |
| 2 | 61371826 | 61372363 | 8 | 67344547 | 67344807 | 17 | 46655103 | 46656182 |
| 2 | 71192000 | 71192568 | 8 | 82192468 | 82192699 | 17 | 75369454 | 75369665 |
| 2 | 79739858 | 79740137 | 8 | 145026044 | 145026218 | 17 | 75370282 | 75370463 |
| 2 | 85811380 | 85811605 | 9 | 71788714 | 71788912 | 18 | 22930220 | 22930423 |
| 2 | 198651301 | 198651522 | 10 | 3824983 | 3825205 | 18 | 32847504 | 32847644 |
| 2 | 202122469 | 202122655 | 10 | 4868452 | 4868692 | 18 | 63417383 | 63417847 |

(continued)

| Chrom osome Numbe ring | Starting Coordinate | Ending Point Coordinate | Chrom osome Numbe ring | Starting Coordinate | Ending Point Coordinate | Chrom osome Numbe ring | Starting Coordinate | Ending Point Coordinate |
|---|---|---|---|---|---|---|---|---|
| 2 | 219735814 | 219736484 | 10 | 7708613 | 7708807 | 18 | 67067675 | 67068447 |
| 2 | 238583502 | 238583678 | 10 | 11059980 | 11060219 | 19 | 18811728 | 18811959 |
| 3 | 107318030 | 107318141 | 10 | 93999632 | 93999840 | 19 | 24270071 | 24270250 |
| 3 | 124860555 | 124861047 | 10 | 103875120 | 103875157 | 19 | 37329224 | 37329450 |
| 3 | 145968575 | 145969138 | 10 | 104433954 | 104434182 | 19 | 39360409 | 39360643 |
| 4 | 8200823 | 8201296 | 10 | 114136009 | 114136222 | 19 | 45901589 | 45901813 |
| 4 | 20253867 | 20254101 | 11 | 64993204 | 64993419 | 19 | 48833393 | 48833741 |
| 4 | 39045787 | 39045942 | 11 | 112833713 | 112833949 | 19 | 48918114 | 48918285 |
| 4 | 42153636 | 42153877 | 11 | 113929754 | 113929967 | 19 | 49127279 | 49127709 |
| 4 | 57522434 | 57522803 | 12 | 15114451 | 15114496 | 19 | 56904839 | 56905081 |
| 4 | 122301671 | 122302189 | 12 | 30975737 | 30976018 | 19 | 57106536 | 57106718 |
| 4 | 170947181 | 170947417 | 12 | 111471428 | 111472512 | 22 | 17082523 | 17082761 |
| 4 | 174430553 | 174430889 | 12 | 111842870 | 111843076 | 22 | 24236256 | 24236465 |
| 5 | 2038682 | 2039002 | 12 | 121570748 | 121570895 | 22 | 40390895 | 40391093 |
| 5 | 42992713 | 42992956 | 13 | 28498274 | 28499128 | | | |
| 5 | 74965037 | 74965188 | 13 | 28501124 | 28501300 | | | |

Based on 103 TOO-related DMR regions, origin evaluation was performed on 473 six-cancer-positive samples in independent Validation set 2. The accuracy of the first origin was 63.0% (298/473), and the accuracy could be improved to 71.5% (338/473) if the sub-optimal origin results were included.

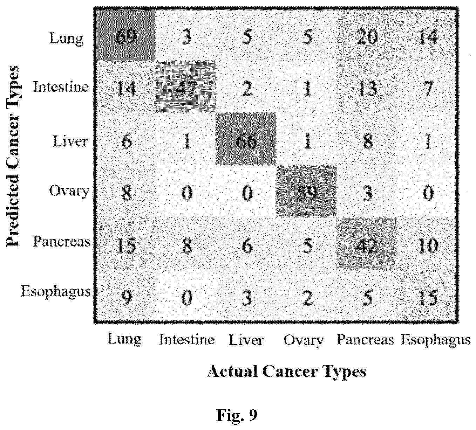

Fig. 9 shows the results of the tissue of origin evaluation based on 103 TOO-related DMR regions.

Four rounds of repeated tests were then conducted, for each adopting random 50 out of the 103 TOO regions, and the origin accuracy results in the four rounds of evaluation are shown in the Table below:

| | Replicate 1 | Replicate 2 | Replicate 3 | Replicate 4 | Replicate 5 |
|---|---|---|---|---|---|
| Number of Accurate Cases of the First Origin/Number of Positive Cases | 56.0% | 55.8% | 50.5% | 58.6% | 56.0% |
| Number of Accurate Cases of the First or Second Origin/Number of Positive Cases | 68.3% | 67.0% | 67.9% | 67.9% | 69.3% |

## Example 6

Results for 222 DMRs evaluated simultaneously for DOC and TOO:
Table 1C shows the 222 DMR regions adopted for DOC and TOO evaluation models.

| Chrom osome Numbe ring | Starting Coordinate | Ending Point Coordinate | Chrom osome Numbe ring | Starting Coordinate | Ending Point Coordinate | Chrom osome Numbe ring | Starting Coordinate | Ending Point Coordinate |
|---|---|---|---|---|---|---|---|---|
| 1 | 12655194 | 12655421 | 6 | 24358259 | 24358357 | 14 | 105714537 | 105715205 |
| 1 | 16482493 | 16482728 | 6 | 42072089 | 42072326 | 15 | 37170394 | 37170556 |
| 1 | 36042931 | 36043301 | 6 | 43252804 | 43253017 | 15 | 41787759 | 41787950 |

(continued)

| | | | | | | | | |
|---|---|---|---|---|---|---|---|---|
| 1 | 57889542 | 57889783 | 6 | 74290168 | 74290371 | 15 | 45479588 | 45479962 |
| 1 | 66258710 | 66258919 | 6 | 78173672 | 78173887 | 15 | 48937168 | 48937477 |
| 1 | 94702665 | 94702906 | 6 | 84418729 | 84418959 | 15 | 64456031 | 64456090 |
| 1 | 111217138 | 111217787 | 6 | 136358883 | 136359048 | 15 | 65134265 | 65134456 |
| 1 | 158151015 | 158151221 | 7 | 3341470 | 3341704 | 15 | 65186260 | 65186486 |
| 1 | 198651112 | 198651193 | 7 | 4923086 | 4923319 | 15 | 73989524 | 73989576 |
| 1 | 198904137 | 198904342 | 7 | 27191055 | 27191278 | 15 | 101513738 | 101513975 |
| 1 | 206680198 | 206680422 | 7 | 27195859 | 27196998 | 16 | 103520 | 103731 |
| 1 | 214156143 | 214156385 | 7 | 37487553 | 37487794 | 16 | 21831585 | 21831778 |
| 1 | 223302649 | 223303032 | 7 | 37956219 | 37956453 | 16 | 22019277 | 22019517 |
| 1 | 226288395 | 226288534 | 7 | 86273683 | 86273892 | 16 | 29819033 | 29819240 |
| 1 | 226867905 | 226868009 | 7 | 87230150 | 87230511 | 16 | 29887965 | 29888205 |
| 1 | 226925137 | 226925338 | 7 | 87848665 | 87848853 | 16 | 54970257 | 54970478 |
| 1 | 228194384 | 228194584 | 7 | 99517220 | 99517409 | 16 | 56692015 | 56692229 |
| 1 | 228645426 | 228645690 | 8 | 37655799 | 37656022 | 16 | 86527879 | 86528007 |
| 1 | 240255077 | 240255308 | 8 | 49468682 | 49468996 | 16 | 88496952 | 88497148 |
| 1 | 243646393 | 243646583 | 8 | 67344547 | 67344807 | 17 | 6659010 | 6659487 |
| 2 | 1747021 | 1748795 | 8 | 82192468 | 82192699 | 17 | 10101537 | 10101760 |
| 2 | 29337908 | 29338440 | 8 | 110986318 | 110987163 | 17 | 14204878 | 14205115 |
| 2 | 30371367 | 30371514 | 8 | 121822423 | 121822624 | 17 | 26699107 | 26699333 |
| 2 | 54786104 | 54786165 | 8 | 145026044 | 145026218 | 17 | 27893085 | 27893267 |
| 2 | 61371826 | 61372363 | 9 | 71788714 | 71788912 | 17 | 27940345 | 27940720 |
| 2 | 66653206 | 66653434 | 9 | 71789479 | 71789804 | 17 | 29298094 | 29298735 |
| 2 | 70313362 | 70313599 | 9 | 100747421 | 100747647 | 17 | 33390708 | 33390856 |
| 2 | 71192000 | 71192568 | 10 | 3824983 | 3825205 | 17 | 38347541 | 38347979 |
| 2 | 74782034 | 74782259 | 10 | 4868452 | 4868692 | 17 | 38478748 | 38478962 |
| 2 | 79739858 | 79740137 | 10 | 7451768 | 7453113 | 17 | 40439431 | 40439630 |
| 2 | 85804601 | 85804852 | 10 | 7708613 | 7708807 | 17 | 46655103 | 46656182 |
| 2 | 85811380 | 85811605 | 10 | 11059980 | 11060219 | 17 | 72322101 | 72322336 |
| 2 | 127413777 | 127414548 | 10 | 15761442 | 15762330 | 17 | 72348118 | 72348323 |
| 2 | 127977497 | 127977735 | 10 | 26727259 | 26727770 | 17 | 73749617 | 73749864 |
| 2 | 175594890 | 175595121 | 10 | 35930440 | 35930678 | 17 | 75369454 | 75369665 |
| 2 | 176931652 | 176933468 | 10 | 65029113 | 65029339 | 17 | 75370282 | 75370463 |
| 2 | 198651301 | 198651522 | 10 | 93999632 | 93999840 | 18 | 7568126 | 7568973 |
| 2 | 202122469 | 202122655 | 10 | 94821612 | 94821821 | 18 | 22930220 | 22930423 |
| 2 | 208492002 | 208492183 | 10 | 103875120 | 103875157 | 18 | 32847504 | 32847644 |
| 2 | 219735814 | 219736484 | 10 | 104433954 | 104434182 | 18 | 43652112 | 43652345 |
| 2 | 238583502 | 238583678 | 10 | 114136009 | 114136222 | 18 | 63417383 | 63417847 |
| 3 | 96532772 | 96533661 | 10 | 116286372 | 116286976 | 18 | 67067675 | 67068447 |
| 3 | 107318030 | 107318141 | 10 | 124220675 | 124220907 | 19 | 13210296 | 13210526 |

(continued)

| | | | | | | | | |
|---|---|---|---|---|---|---|---|---|
| 3 | 124860555 | 124861047 | 10 | 134121362 | 134121537 | 19 | 15090186 | 15090459 |
| 3 | 128336534 | 128337139 | 11 | 14926677 | 14927170 | 19 | 18811728 | 18811959 |
| 3 | 142838826 | 142839024 | 11 | 30607011 | 30607243 | 19 | 20277961 | 20278146 |
| 3 | 145968575 | 145969138 | 11 | 64993204 | 64993419 | 19 | 23299837 | 23300068 |
| 3 | 147129890 | 147130046 | 11 | 112833713 | 112833949 | 19 | 24270071 | 24270250 |
| 3 | 169382104 | 169382265 | 11 | 113929754 | 113929967 | 19 | 36909358 | 36909593 |
| 3 | 192125963 | 192126200 | 11 | 120856596 | 120856827 | 19 | 37329224 | 37329450 |
| 4 | 8200823 | 8201296 | 12 | 4273745 | 4273938 | 19 | 39360409 | 39360643 |
| 4 | 16084194 | 16084859 | 12 | 4378216 | 4378485 | 19 | 45901589 | 45901813 |
| 4 | 20253867 | 20254101 | 12 | 14134428 | 14134635 | 19 | 46012638 | 46012867 |
| 4 | 39045787 | 39045942 | 12 | 15114451 | 15114496 | 19 | 48833393 | 48833741 |
| 4 | 42153636 | 42153877 | 12 | 25055909 | 25056424 | 19 | 48918114 | 48918285 |
| 4 | 57522434 | 57522803 | 12 | 30975737 | 30976018 | 19 | 49127279 | 49127709 |
| 4 | 96469517 | 96471053 | 12 | 53108103 | 53108332 | 19 | 54485996 | 54486235 |
| 4 | 104641262 | 104641323 | 12 | 63025682 | 63026599 | 19 | 54926374 | 54926594 |
| 4 | 122301671 | 122302189 | 12 | 99288744 | 99288978 | 19 | 56904839 | 56905081 |
| 4 | 170947181 | 170947417 | 12 | 111471428 | 111472512 | 19 | 57078725 | 57078948 |
| 4 | 174430553 | 174430889 | 12 | 111842870 | 111843076 | 19 | 57106536 | 57106718 |
| 5 | 2038682 | 2039002 | 12 | 113344913 | 113344985 | 20 | 3073502 | 3073739 |
| 5 | 17216866 | 17217095 | 12 | 121570748 | 121570895 | 20 | 24449663 | 24450083 |
| 5 | 42992713 | 42992956 | 12 | 122519672 | 122519821 | 20 | 34189352 | 34189582 |
| 5 | 74965037 | 74965188 | 13 | 27334667 | 27334905 | 20 | 39958232 | 39958425 |
| 5 | 76011073 | 76011622 | 13 | 28498274 | 28499128 | 20 | 46414877 | 46415116 |
| 5 | 123985317 | 123985370 | 13 | 28501124 | 28501300 | 20 | 58514747 | 58514974 |
| 5 | 139047953 | 139048324 | 13 | 28502583 | 28503595 | 21 | 26934381 | 26934887 |
| 5 | 139227564 | 139227787 | 13 | 61987902 | 61988077 | 21 | 28216998 | 28217857 |
| 5 | 140797161 | 140797369 | 14 | 30396461 | 30396694 | 21 | 47518006 | 47518240 |
| 5 | 142781721 | 142781863 | 14 | 50527870 | 50528014 | 22 | 17082523 | 17082761 |
| 5 | 167956245 | 167956565 | 14 | 59931484 | 59932323 | 22 | 24236256 | 24236465 |
| 5 | 173345668 | 173345841 | 14 | 85998437 | 85998850 | 22 | 40390895 | 40391093 |
| 6 | 10884092 | 10884316 | 14 | 89817960 | 89818103 | 22 | 51112158 | 51112388 |

In the independent Validation set, sensitivity and origin accuracy were calculated at a unified specificity of 95.1% (450/473) for 473 negative samples and 473 positive six-cancer-samples under condition with a marker number of 222. The tumor detection and tissue origin results assessed are shown in the Table below:

| Cancer Types | Lung Carcinoma | Colorectal Carcinoma | Hepatocellular Carcinoma | Ovarian Carcinoma | Pancreatic Adenocarcinoma | Esophageal Carcinoma |
|---|---|---|---|---|---|---|
| Total Number of Cases | 121 | 59 | 82 | 73 | 91 | 47 |

(continued)

| Cancer Types | | Lung Carcinoma | Colorectal Carcinoma | Hepatocellular Carcinoma | Ovarian Carcinoma | Pancreatic Adenocarcinoma | Esophageal Carcinoma |
|---|---|---|---|---|---|---|---|
| 222 DMRs | Number of Positive Cases | 63 | 29 | 63 | 64 | 38 | 25 |
| | Sensibility | 52.1% | 49.2% | 76.8% | 87.7% | 41.8% | 53.2% |
| | Number of Cases with Accurate Detection and Origin | 39 | 25 | 58 | 59 | 29 | 17 |
| | Accuracy of Detection and Origin | 32.2% | 42.4% | 70.7% | 80.8% | 31.9% | 36.2% |

| Total Number of Cases | Number of Positive Cancer Cases | Overall Sensitivity for Cancer | Number of Accurate Cases of the First Origin | Accuracy of the First Origin | Number of Accurate Cases of the First or Sub-optimal Origin | Accuracy of the First or Sub-optimal Origin |
|---|---|---|---|---|---|---|
| 222 DMRs | 282 | 59.6% | 229 | 81.2% | 248 | 87.9% |

The foregoing detailed description is provided by way of explanation and illustration, and is not intended to limit the scope of the appended claims. The numerous variations of the embodiments presently recited in the present application will be apparent to those skilled in the art and remain within the scope of the appended claims and equivalents thereof.

**Claims**

1. A method for evaluating correlation between a sample to be tested and risk of tumor formation and/or tumor tissue of origin, **characterized by** comprising: (1) a differentially methylated region DMR classification step: determining a plurality of target DMRs for evaluation, based on sequencing coverage depth of a methylated site and/or methylation level difference of adjacent methylated sites; (2) a tumor formation risk evaluation step: evaluating the correlation between the sample to be tested and the risk of tumor formation, based on methylation levels of the target DMRs of the sample to be tested; (3) optionally comprising a tumor tissue of origin evaluation step: evaluating the correlation between the sample to be tested and the tumor tissue of origin, based on methylation levels of the target DMRs of the sample to be tested.

2. A method for determining a differentially methylated region DMR, **characterized by** the method comprising a differentially methylated region DMR classification step: determining a differentially methylated region DMR, based on sequencing coverage depth of a methylated site and/or methylation level difference of adjacent methylated sites.

3. A method for evaluating correlation between a sample to be tested and risk of tumor formation, **characterized by** comprising a tumor formation risk evaluation step: evaluating the correlation between the sample to be tested and the risk of tumor formation, based on methylation level of the DMR of the sample to be tested, wherein the method comprising a step of reducing influence of age factor of a subject on evaluation result, wherein the sample to be tested is derived from the subject.

4. A method for evaluating correlation between a sample to be tested and tumor tissue of origin, **characterized by** comprising a tumor tissue of origin evaluation step: evaluating the correlation between the sample to be tested and the tumor tissue of origin, based on methylation level of the DMR of the sample to be tested through a multi-classification method and logistic regression.

5. The method of any one of claims 1-4, comprising determining an absolute value of methylation level difference between a methylated site and an adjacent methylated site thereof, and determining whether the methylated site and the adjacent methylated site thereof being classified into the same DMR based on the absolute value of the difference.

6. The method of claim 5, comprising determining a weight for the absolute value of the difference, wherein the weight for the absolute value of the difference being determined according to the sequencing coverage depth of the methylated site.

7. The method of any one of claims 1-6, wherein the methylation level difference $\beta_{ij}$ is determined according to the following formula:

$$\beta_{ij} = \left| M_{ij} - M_{i\,(j+1)} \right| * e^{(1-P_{ij})}$$

wherein $M_{ij}$ is the methylation level of the ith sample at the jth site, e represents the natural constant, and $P_{ij}$ is determined according to the following formula:

$$P_{ij} \propto \frac{1}{e^{\frac{|d_{ij} - d_{i(j+1)}|}{d_{ij} + d_{i(j+1)}}}}$$

wherein $d_{ij}$ is the sequencing coverage depth of the ith sample at the jth site.

8. The method of claim 7, wherein the methylated site and the adjacent methylated site thereof are determined to be classified into the same DMR when the methylation level difference $\beta_{ij}$ of the methylated sites is less than or equal to about 0.25.

9. The method of any one of claims 1-8, further comprising determining a degree of fluctuation of methylation level of the DMR, based on the difference in methylation levels of a methylated site inside the DMR and a methylated site at intermediate position of the DMR.

10. The method of claim 9, wherein the degree of fluctuation of methylation level of the DMR $B_{ij}$ is determined according to the following formula:

$$B_{ij} = \frac{1}{n} \sum_{j=1}^{n} \frac{|\beta_{ij} - \mu_j|}{\left| \beta_{ij} - \beta_{i\,(j+1)} \right|}$$

wherein $\beta_{ij}$ is the methylation level difference at the jth site of the ith sample, and $\mu_j$ is the methylation level difference of the methylated site at intermediate position of DMR region.

11. The method of claim 10, wherein determining a DMR with $B_{ij}$ less than about 1 being adopted for evaluating the correlation between the sample to be tested and the risk of tumor formation and/or tumor tissue of origin.

12. The method according to any one of claims 1-11, comprising evaluating the correlation between the sample to be tested and the risk of tumor formation through a binary classification model, based on the methylation level of DMR of the sample to be tested, wherein the evaluating method reduces influence of age factor of a subject on evaluation result of the correlation between the sample to be tested and the risk of tumor formation, wherein the sample to be tested is derived from the subject.

13. The method of claim 12, wherein the binary classification model comprises a support vector machine SVM model.

14. The method of claim 13, comprising introducing a penalty term based on the age factor into the SVM model.

15. The method of claim 14, comprising introducing a penalty term based on the age factor in the SVM model by way of Hilbert-Schmidt independence criterion.

16. The method of any one of claims 1-15, comprising preforming machine learning training for training samples known to have tumor formation or known to be free of tumor formation according to the following formula:

$$f(\mathrm{x}; \mathrm{w}, \mathrm{b}) = sgn\ (wT\mathrm{x} + b)$$

if a < 0, sgn (a) = -1
if a ≥ 0, sgn (a) = 1 and the following equation being adopted to determine training parameters:

$$\min_{w \in R^n, b \in R, \xi \in R^m} \frac{1}{2} wT w + C \sum_{i=1}^{m} \xi_i + \lambda L_H \left( P_{h(x)h(z)} \right)$$

s.t.

$$y_i(wT\mathrm{x} + b) \geq 1 - \xi_i$$

$$\xi_i \geq 0$$

wherein $C$, w, $\lambda$, b represent training parameters, sgn() represents sign function, $\xi_i$ represents degree to which sample $x_i$ violates the equation, x represents methylation level of a sample, y represents as +1 when a sample is correlated with tumor formation, y represents as -1 when a sample is not correlated with tumor formation, and $L_H(P_{h(x)h(z)})$ being determined by the following formula:

$$L_H \left( P_{h(x)h(z)}, F, G \right) := \left\| C_{h(x)h(z)} \right\|_{HS}^2$$

$$\left\| C_{h(y)h(z)} \right\|^2 = \left( E_{h(x)h(z)} - E_{h(x)}E_{h(z)} \right)^2$$

$$= \left( E_{h(x)h(z)} \right)^2 + \left( E_{h(x)}E_{h(z)} \right)^2 - 2E_{h(x)h(z)}E_{h(x)}E_{h(z)}$$

wherein $h(y)$ and $h(z)$ are kernel functions of $Y$ and $Z$ respectively, F and G represent the reproducing kernel Hilbert space of $X$ and $Z$ respectively, and $P_{h(x)h(z)}$ represents probability distribution of $h(y)$ and $h(z)$.

17. The method of any one of claims 1-16, comprising evaluating the correlation between the sample to be tested and the tumor tissue of origin through determining classification probabilities by a multi-classification method, and fitting the classification probabilities by logistic regression, based on methylation level of the DMR of the sample to be tested.

18. The method of claim 17, wherein the classification probabilities are determined by pairwise voting of binary classification.

19. The method of any one of claims 17-18, wherein the classification probabilities are fitted by multiple linear regression MLR.

20. The method of any one of claims 1-19, comprising performing regression analysis on training samples with known tissue of origin according to the following formula:

classification probabilities $p_i^v$ determined by binary classification being determined according to the following formula:

$$p_i^v = 2 \sum_{i:j \neq i} I_{\{r_{ij} > r_{ji}\}} / (k(k-1))$$

$$\mu_{ij} \equiv P(y = i \mid y = i \text{ or } j, x)$$

wherein I (x) is target equation: $I_{\{x\}}$ = 1 if $x$ is true, $I_{\{x\}}$ = -1 if $x$ is false, $r_{ij}$ is an estimate of pairwise classification probability $\mu_{ij}$, k is sum of tissue classes; i and j represent the ith and jth class respectively, and $x$ represents methylation level of the DMR of a sample;

and weight $\beta_j$ for multiple linear regression MLR fitting being determined according to the following formula:

$$E\{Y_{ij}\} = \frac{exp\left(X'_{i} \beta_j\right)}{1 + \sum_{k=1}^{J-1} exp\left(X'_{i} \beta_k\right)} \quad j = 1, 2, \ldots, J - 1$$

wherein $X'_i$ represents classification probabilities obtained by pairwise voting of binary classification, and $Y_{ij}$ represents tissue of origin class of a sample.

21. The method of any one of claims 1-20, wherein tissue of origin of the training samples being corrected based on probability that the sample has tumor formation.

22. The method of claim 21, comprising performing the correction after the pairwise voting of binary classification obtaining classification probabilities results and before the multiple linear regression analysis.

23. The method of any one of claims 21-22, comprising performing the correction based on a quasi-maximum likelihood estimation method.

24. The method of any one of claims 21-23, comprising performing the correction according to the following formula:

$$\prod_{i=1}^{n} f_i(y_i)^{w_i} = \prod_{i=1}^{n} \pi_i^{y_i w_i} (1 - \pi_i)^{(1-y_i)w_i}$$

wherein $y_i$ represents tissue of origin class of a sample, $w_i$ represents weight of the correction, $\pi_i$ represents probability that the sample has tumor formation.

25. A storage medium, recording a program capable of operating the method according to any one of claims 1-24.

26. An apparatus, wherein the apparatus comprising the storage medium of claim 25, and optionally comprising a processor coupled to the storage medium, and the processor being configured to implement the method of any one of claims 1-24 by executing based on a program stored in the storage medium.

27. A system for evaluating correlation between a sample to be tested and risk of tumor formation and/or tumor tissue of origin, **characterized by** comprising: (1) a differentially methylated region DMR classification module: used for determining a plurality of target DMRs for evaluation, based on sequencing coverage depth of a methylated site and/or methylation level difference of adjacent methylated sites; (2) a tumor formation risk evaluation module: used for evaluating the correlation between the sample to be tested and the risk of tumor formation, based on methylation levels of the target DMRs of the sample to be tested; (3) optionally comprising a tumor tissue of origin evaluation module: used for evaluating the correlation between the sample to be tested and the tumor tissue of origin, based on methylation levels of the target DMRs of the sample to be tested.

28. A system used for determining a differentially methylated region DMR, **characterized by** comprising a differentially methylated region DMR classification module: used for determining a differentially methylated region DMR, based on sequencing coverage depth of a methylated site and/or methylation level difference of adjacent methylated sites.

29. A system for evaluating correlation between a sample to be tested and risk of tumor formation, **characterized by**

comprising a tumor formation risk evaluation module: used for evaluating the correlation between the sample to be tested and the risk of tumor formation, based on methylation level of the DMR of the sample to be tested, wherein the method comprising a module used for reducing influence of age factor of a subject on evaluation result, wherein the sample to be tested is derived from the subject.

30. A system for evaluating correlation between a sample to be tested and tumor tissue of origin, **characterized by**: comprising a tumor tissue of origin evaluation module: used for evaluating the correlation between the sample to be tested and the tumor tissue of origin, based on methylation level of the DMR of the sample to be tested through a multi-classification method and logistic regression.

Fig. 1

Fig. 2

Fig. 3

|  | | Predicted | | | | | | Sum | Accuracy |
|---|---|---|---|---|---|---|---|---|---|
|  | | LC | CRC | LIHC | OVCA | PAAD | ESCA | Healthy samples | Sum | Accuracy |
| Actual | LC | 44 | | | | | | | 44 | 100% |
|  | CRC | | 32 | | | | | | 32 | 100% |
|  | LIHC | | | 26 | | | | 1 | 27 | 96% |
|  | OVCA | | | | 64 | | | 1 | 67 | 96% |
|  | PAAD | | | | | 67 | | | 70 | 96% |
|  | ESCA | | | | | | 36 | | 40 | 90% |
|  | Healthy samples | | | | | | | 55 | 55 | 100% |
|  | Sum | 44 | 32 | 26 | 66 | 68 | 37 | 57 | 332 | 98% |
|  | PPV | 100% | 100% | 100% | 97% | 99% | 97% | 96% | | |

Fig. 4

Fig. 5

Fig. 6A

Fig. 6B

**Fig. 6C**

**Fig. 6D**

Pancreatic Adenocarcinoma

**Fig. 6E**

Esophageal Carcinoma

**Fig. 6F**

Fig. 7A

Fig. 7B

Age Group

**Fig. 7C**

Age Group

**Fig. 7D**

Fig. 7E

Fig. 7F

EP 4 567 817 A1

Fig. 8

38

Fig. 9

## INTERNATIONAL SEARCH REPORT

| | International application No. |
|---|---|
| | **PCT/CN2022/129143** |

| **A.** | **CLASSIFICATION OF SUBJECT MATTER** |
|---|---|
| | G16B20/20(2019.01)i |

According to International Patent Classification (IPC) or to both national classification and IPC

| **B.** | **FIELDS SEARCHED** |
|---|---|

Minimum documentation searched (classification system followed by classification symbols)

G16B

Documentation searched other than minimum documentation to the extent that such documents are included in the fields searched

Electronic data base consulted during the international search (name of data base and, where practicable, search terms used)

VEN, CNABS, CNTXT, WOTXT, EPTXT, USTXT, CNKI, IEEE: 肿瘤, 癌症, 甲基化, 差异, 风险, 评估, 相关性, tumor, cancer, tissue, evaluat+, different, risk, methylat+

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| PX | CN 115132273 A (GUANGZHOU BURNING ROCK DX LABORATORY CO., LTD.) 30 September 2022 (2022-09-30) description, paragraphs [0004]-[0015], and claims 1-30 | 1-30 |
| X | TW 201718876 A (LAI HONGZHENG) 01 June 2017 (2017-06-01) description, paragraphs [0041]-[0161] | 1-30 |
| A | CN 113539355 A (YUNKANG INFORMATION TECHNOLOGY (SHANGHAI) CO., LTD.) 22 October 2021 (2021-10-22) entire document | 1-30 |
| A | CN 114171115 A (SHENZHEN GENEPLUS MEDICAL SCIENCE EXAMINATION LABORATORY) 11 March 2022 (2022-03-11) entire document | 1-30 |
| A | CN 114736968 A (NANJING SHIHE MEDICAL EQUIPMENT CO., LTD.) 12 July 2022 (2022-07-12) entire document | 1-30 |

☐ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **14 April 2023** | **20 April 2023** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**
Information on patent family members

| International application No. |
| --- |
| **PCT/CN2022/129143** |

| Patent document cited in search report | | | Publication date (day/month/year) | Patent family member(s) | Publication date (day/month/year) |
| --- | --- | --- | --- | --- | --- |
| CN | 115132273 | A | 30 September 2022 | None | |
| TW | 201718876 | A | 01 June 2017 | None | |
| CN | 113539355 | A | 22 October 2021 | None | |
| CN | 114171115 | A | 11 March 2022 | None | |
| CN | 114736968 | A | 12 July 2022 | None | |

Form PCT/ISA/210 (patent family annex) (July 2022)